(19) **Europäisches Patentamt / European Patent Office / Office européen des brevets**

(11) **EP 4 786 577 A1**

(12) **EUROPEAN PATENT APPLICATION**
published in accordance with Art. 153(4) EPC

(43) Date of publication:
05.08.2026 Bulletin 2026/32

(21) Application number: 24869878.9

(22) Date of filing: 21.06.2024

(51) International Patent Classification (IPC):
$C12N\ 1/20^{(2026.01)}$    $A61K\ 35/747^{(2015.01)}$
$A61P\ 3/06^{(2006.01)}$    $A61P\ 31/04^{(2006.01)}$
$C12R\ 1/25^{(2006.01)}$    $C12P\ 7/56^{(2006.01)}$
$A61P\ 3/04^{(2006.01)}$

(52) Cooperative Patent Classification (CPC):
A61K 35/747; A61P 3/04; A61P 3/06; A61P 31/04;
C12N 1/20; C12P 7/56; C12R 2001/25

(86) International application number:
PCT/CN2024/100581

(87) International publication number:
WO 2025/066317 (03.04.2025 Gazette 2025/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 28.09.2023 CN 202311294650
28.09.2023 CN 202311294687

(71) Applicant: COREE Company Limited
Hong Kong 999077 (HK)

(72) Inventors:
• WANG, Tingting
Beijing 101300 (CN)
• ZHANG, Shiqi
Beijing 101300 (CN)
• ZHANG, Xiao
Beijing 101300 (CN)
• ZHAO, Ying
Beijing 101300 (CN)
• FAN, Linlin
Beijing 101300 (CN)
• ZHANG, Zhengwen
Beijing 101300 (CN)
• LEE, Soowon
Beijing 101300 (CN)
• XU, Ying
Beijing 101300 (CN)
• LIM, Chongyoon
Beijing 101300 (CN)

(74) Representative: Studio Torta S.p.A.
Via Viotti, 9
10121 Torino (IT)

Remarks:
The complete document including Reference
Table(s) and the Sequence Listing(s) can be
downloaded from the EPO website

(54) **LACTIPLANTIBACILLUS PLANTARUM STRAIN HAVING WEIGHT-REDUCING FUNCTION, PROBIOTIC FORMULATION, PREPARATION METHOD THEREFOR, AND USE THEREOF**

(57) The present invention particularly relates to a *Lactiplantibacillus plantarum* (*L. plantarum*) strain with weight loss function, a viable bacteria preparation, and a preparation method and use thereof. The *L. plantarum* strain is deposited in China General Microbiological Culture Collection Center (CGMCC) with a deposit number of CGMCC No. 25683. The *L. plantarum* strain and the viable bacteria preparation thereof can inhibit the growth of the obese strain *Enterobacter cloacae*, reduce cholesterol, inhibit the differentiation of 3T3-L1 preadipocytes, reduce the body weight of obesity model mice, reduce the total weight gain and the total food utilization rate of obesity model rats, and have excellent weight loss function.

EP 4 786 577 A1

FIG. 8

**Description**

**CROSS-REFERENCE TO RELATED APPLICATIONS**

This application claims priority to Chinese Patent Application No. 202311294650.7 filed in China on September 28, 2023 and Chinese Patent Application No. 202311294687.X filed in China on September 28, 2023, the entire contents of which are incorporated herein by reference.

**TECHNICAL FIELD**

The present disclosure relates to the technical field of microorganisms, in particular to a *Lactiplantibacillus plantarum (L. plantarum)* strain with weight loss function, a probiotic preparation, and a preparation method therefore and use thereof.

**BACKGROUND**

Obesity is mainly a metabolic disease caused by disorder of the energy metabolism balance of the body, resulting in energy intake greater than energy consumption, and thus excessive accumulation of fat in the body. Obesity is not only affected by multiple factors such as genetics, environment, metabolism, and physiology, but also closely related to diseases such as hyperlipidemia, hypertension, diabetes, insulin resistance, hyperuricemia, non-alcoholic fatty liver disease, and coronary atherosclerotic heart disease. Obesity is a global public health problem, according to statistics, that one-third of the world's population is overweight, the obesity rate of adults has increased by 50% in the past 20 years, and children and adolescents obesity are doubled. According to the latest data of "Report on the Nutrition and Chronic Disease Status of Chinese Residents (2020)", there have been over 1/2 overweight or obesity in adults in the country. The overweight rate of adult residents ($\geq$ 18 years old) is 34.3% and the obesity rate is 16.4%. Over the past 30 years, the overweight and obesity rates have been increased continuously at all ages, with an average increase of about 2.5 times.

Probiotics can reduce weight by regulating intestinal flora, improving intestinal inflammation, reducing cholesterol content, regulating hormone level, etc. Probiotic for weight loss has the characteristics of zero side effects, zero rebound, no water loss, no skin damage, no dieting, no exercise, simple and convenient use, etc., making weight loss enter a new stage.

In the related art, it is reported that a *L. plantarum* strain isolated from Korea kimchi has a weight loss effect, but the weight loss effect is poor and cannot meet the industrial needs.

Therefore, there is a need for a *L. plantarum* strain with excellent weight loss function and a probiotic preparation comprising the same.

**SUMMARY**

The present invention provides a *L. plantarum* strain with weight loss function, a probiotic preparation comprising the strain, and a preparation method therefore and use thereof, wherein the strain and the probiotic preparation thereof are capable of producing lactic acid and short-chain fatty acids with high yield; are capable of inhibiting various pathogenic bacteria; are capable of adhering to intestinal epithelial cells; are capable of inhibiting the growth of the obese strain *Enterobacter cloacae* (*E. cloacae*); and are capable of inhibiting the differentiation of 3T3-L1 preadipocytes, thereby achieving the effect of effectively inhibiting fat deposition; and are capable of reducing the content of cholesterol.

In order to achieve the above object, the present invention provides the following technical solutions:

In one aspect, the present invention provides a *Lactiplantibacillus plantarum* (*L. plantarum*) strain, the *L. plantarum* strain is deposited in China General Microbiological Culture Collection Center (CGMCC) with a deposit number of CGMCC No. 25683.

Preferably, the *L. plantarum* strain comprises a 16S rRNA gene represented by SEQ ID NO: 1.

In another aspect, the present invention provides a method for isolating the *L. plantarum* strain described above, comprising: taking a suitable sample to proliferate in a suitable medium, then treating it with artificial gastric juice and artificial intestinal juice before isolation.

Preferably, the *L. plantarum* strain is isolated from healthy human breast milk.

In another aspect, the present invention provides a probiotic preparation, which is a viable bacteria preparation or a dead bacteria preparation, and comprises the *L. plantarum* strain described above.

Preferably, the probiotic preparation is a viable bacteria preparation, and the viable bacteria content is 6 to 9 $\times$ $10^{11}$ CFU/g.

Preferably, the probiotic preparation is a dead bacteria preparation, and comprises 7 to 13 $\times$ $10^{11}$ CFU/g of dead bacteria.

In another aspect, the present invention provides a method for preparing the probiotic preparation described above, comprising the following steps:

(1) strain culturing: inoculating the *L. plantarum* strain into a sterile liquid medium at an inoculation amount of 1-3% based on the total amount of the medium, culturing for 16 to 24 hours to obtain a seed culture solution, then inoculating the obtained seed culture solution into a fermentation medium at an inoculation amount of 1-3% based on the total amount of the medium for fermentation culture to obtain a high-density culture solution of *L. plantarum*;
(2) drying.

Preferably, the fermentation medium used comprises: glucose 60-100 g/L, yeast extract 60-80 g/L, magnesium sulfate 0.2-0.4 g/L, manganese sulfate 0.1-0.2 g/L, dipotassium hydrogen phosphate 2-4 g/L, potassium dihydrogen phosphate 2-4 g/L, Tween-80 1-2 g/L, and L-cysteine salt 0.5-2 g/L..

Preferably, during the fermentation, the sodium hydroxide solution is automatically added to maintain a constant pH of 5.0-6.0, so that the fermentation is terminated until the acid production is stopped, and the fermentation is terminated when the sodium hydroxide is no longer added.

In an example, the probiotic preparation is a viable bacteria preparation, and the method further comprises preparing a lyoprotectant after the strain culturing step and before the drying step, and performing the freeze-drying in the drying step; optionally, the lyoprotectant used comprises: protectant of 50-100 g/L peptone, 20-60 g/L sucrose, 5-10 g/L vitamin C and 1-3 g/L glycerol.

Preferably, the conditions of freeze-drying are: a pre-freezing temperature of -50 to -55 °C with pre-freezing time of 5-8 hours, a primary drying temperature of -10 to -30 °C with primary drying time of 25 - 35 hours, and a secondary drying temperature of 32 to 35 °C with secondary drying time of 5-8 hours.

In another example, the probiotic preparation is a dead bacteria preparation, and the drying step is to perform heat inactivation first, and then drying through a spray drying tower to obtain the dead bacteria preparation; optionally, the heat inactivation is performed at 70 to 100 °C for 10 to 40 minutes.

In another aspect, the present invention provides use of the *L. plantarum* strain described above for the production of lactic acid and short-chain fatty acids.

Preferably, the short-chain fatty acid is selected from the group consisting of one or more of formic acid, acetic acid, propionic acid, butyric acid and isobutyric acid.

In another aspect, the present invention provides use of the *L. plantarum* strain described above in the preparation of a composition for inhibiting the obese strain *E. cloacae*.

In another aspect, the present invention provides use of the *L. plantarum* strain described above in the preparation of a composition for inhibiting the differentiation of 3T3-L1 preadipocytes, thereby effectively inhibiting fat deposition.

In another aspect, the present invention provides use, as described above, in the preparation of a pharmaceutical composition for weight loss or reducing hyperlipidemia and hypercholesterolemia caused by obesity.

Preferably, the weight loss function is to reduce the content of cholesterol, or inhibit the differentiation of 3T3-L1 preadipocytes, thereby inhibiting the generation of fat.

Preferably, the weight loss function is one or more selected from the group consisting of reducing body weight, total weight gain and total food utilization rate in obesity model rats; reducing body fat weight and fat/body weight ratio in obesity model rats; increasing serum high-density lipoprotein cholesterol content; increasing intestinal short-chain fatty acid content; and reducing the expression level of cellular inflammatory factor TNF-$\alpha$ in serum.

Preferably, the weight loss function is one or more selected from the group consisting of reducing cholesterol content, inhibiting differentiation of 3T3-L1 precursor adipocytes, reducing body weight of obesity model mice, reducing total weight gain and total food utilization rate in obesity model rats.

In one embodiment, the *L. plantarum* strain according to the present invention can be prepared into ordinary food, health food or medicine.

The beneficial effects of the *L. plantarum* strain and the preparation thereof of the present invention are as follows:

1. The *L. plantarum* strain and the probiotic preparation thereof of the present invention can tolerate artificial gastrointestinal fluids, can produce lactic acid and short-chain fatty acids with high yield, can inhibit various pathogenic bacteria, and can adhere to intestinal epithelial cells.
2. The *L. plantarum* strain and the probiotic preparation thereof of the present invention inhibit the growth of the obese strain *E. cloacae*, and can inhibit the differentiation of 3T3-L1 preadipocytes, thereby achieving the effect of effectively inhibiting fat deposition.
3. The *L. plantarum* strain and the preparation thereof of the present invention can reduce the content of cholesterol.
4. The *L. plantarum* strain and the preparation thereof and the probiotic preparation thereof of the present invention can significantly reduce the body weight of the obesity model rats, reduce the total weight gain and the total food utilization rate of the obesity model rats, significantly reduce the body fat weight and the fat/body ratio of the obesity model rats, increase the content of serum high-density lipoprotein cholesterol, effectively increase the content of intestinal short-chain fatty acids, and reduce the expression level of inflammatory factor TNF- $\alpha$ in serum. It can significantly reduce the body weight of obesity model mice, and reduce the total weight gain and total food utilization of obesity model rats. Therefore, the *L. plantarum* strain of the present invention has excellent weight loss function.

5. The probiotic preparation of the present invention has simple production process parameters, is easy to control, and has a short cycle, which ensures a high survival rate of the *L. plantarum* strain, and the obtained product can be stored for a long time, and the product quality is stable.

## BRIEF DESCRIPTION OF THE DRAWINGS

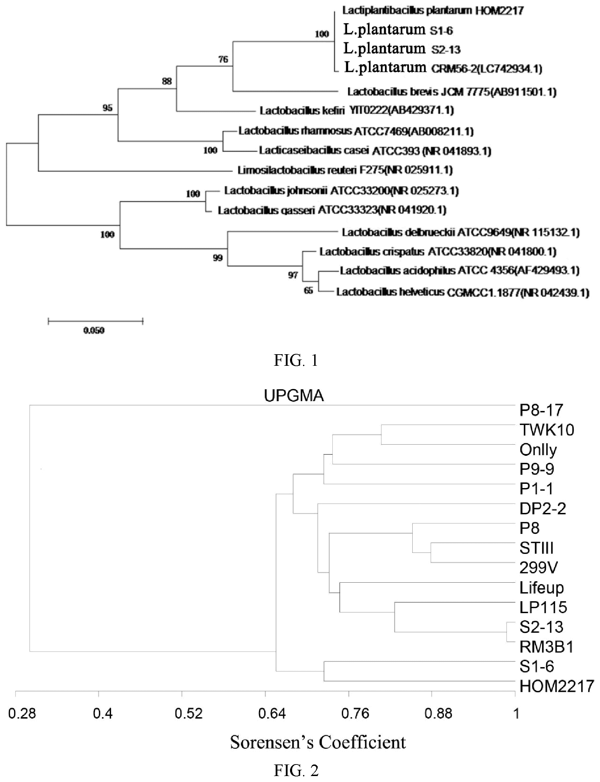

FIG. 1 shows a phylogenetic tree of *L. plantarum* HOM2217 according to the present invention.

FIG. 2 shows a RAPD cluster analysis diagram of *L. plantarum* HOM2217 constructed based on the UPGMA method.

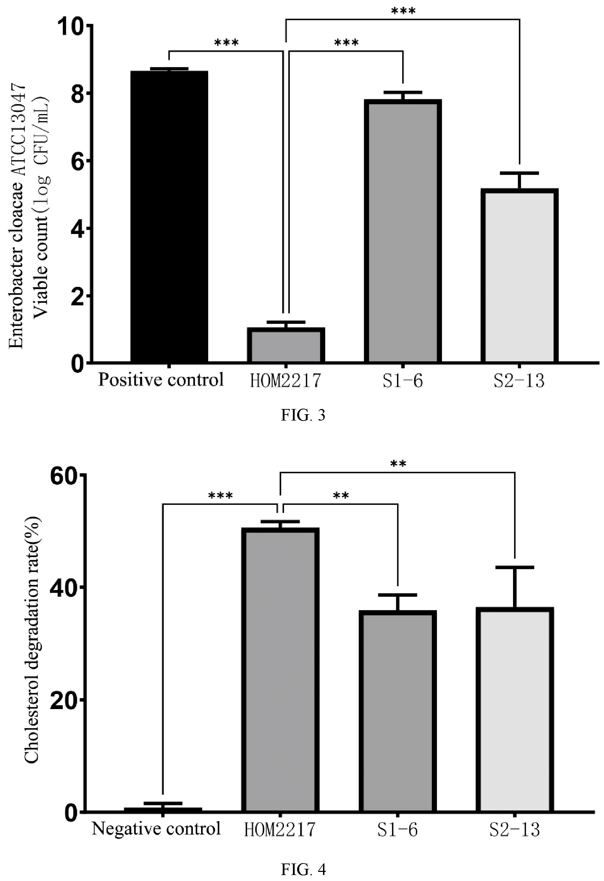

FIG. 3 shows the results of growth inhibition of *E. cloacae* ATCC13047 by *L. plantarum* HOM2217 according to the present invention. Note: *** indicates $p < 0.001$ compared with the HOM2217 group.

FIG. 4 shows the in vitro cholesterol degradation rate of *L. plantarum* HOM2217 according to the present invention. Note: *** indicates $p < 0.001$ compared with the HOM2217 group, and ** represents $p < 0.01$ compared with the HOM2217 group.

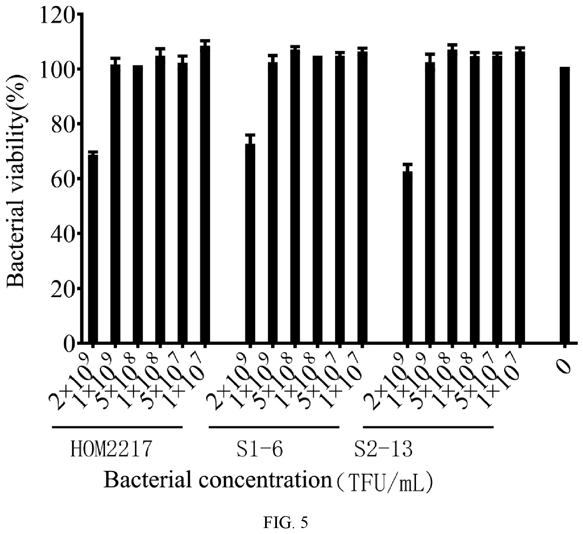

FIG. 5 shows the effect of *L. plantarum* HOM2217 according to the present invention on 3T3-L1 preadipocytes viability.

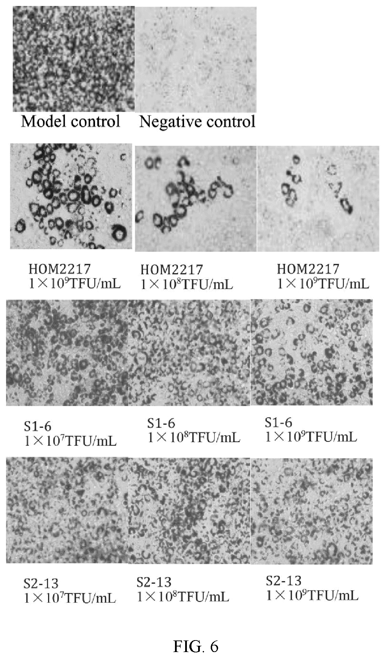

FIG. 6 shows the results of oil red staining of 3T3-L1 preadipocytes differentiation by *L. plantarum* HOM2217 according to the present invention.

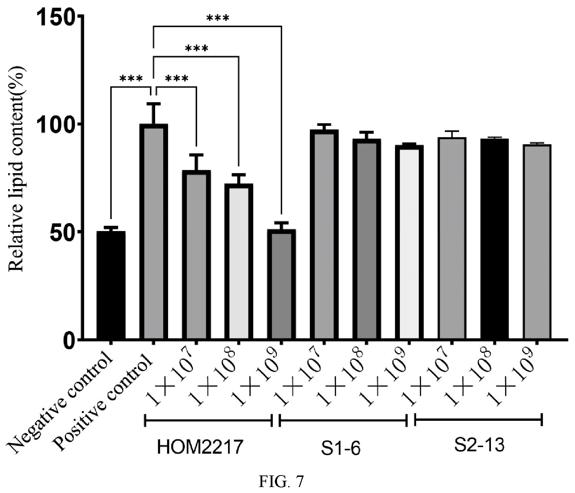

FIG. 7 shows the effect of *L. plantarum* HOM2217 according to the present invention on the lipid content after differentiation of 3T3-L1 preadipocytes.

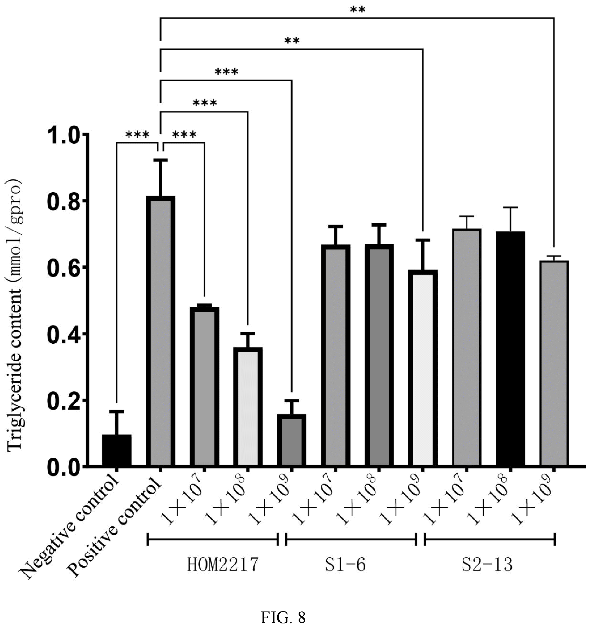

FIG. 8 shows the effect of *L. plantarum* HOM2217 according to the present invention on triglyceride (TG) content after differentiation of 3T3-L1 preadipocyte.

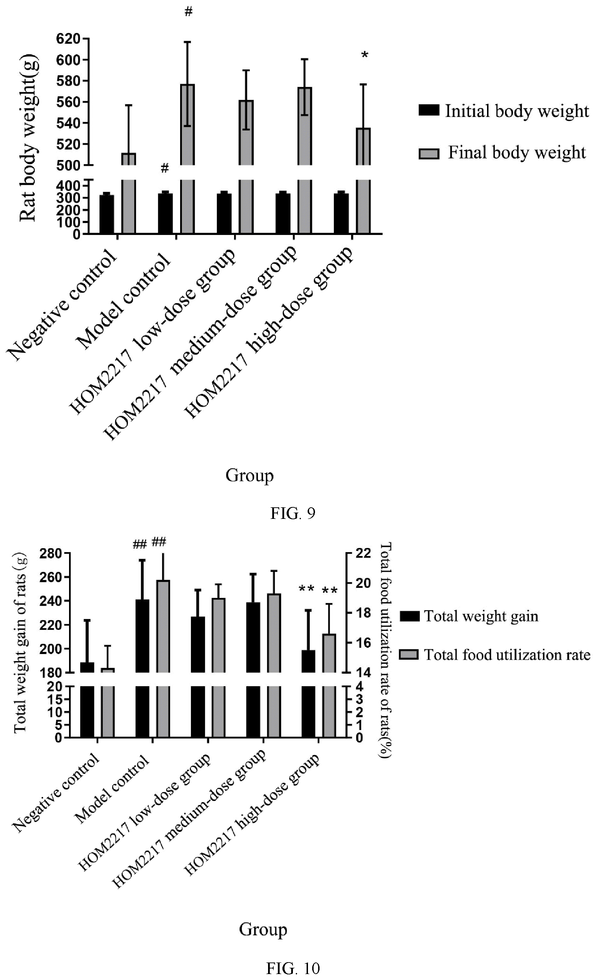

FIG. 9 shows the effect of *L. plantarum* HOM2217 according to the present invention on body weight of obesity model rats. Note: # indicates a significant difference compared with the negative control group ($p < 0.05$); * indicates a significant difference compared with the model control group ($p < 0.05$).

FIG. 10 shows the effect of *L. plantarum* HOM2217 according to the present invention on the total weight gain and total food utilization rate of obesity model rats. Note: ## indicates an extremely significant difference from the negative control group ($p < 0.01$); ** indicates an extremely significant difference from the model control group ($p < 0.01$).

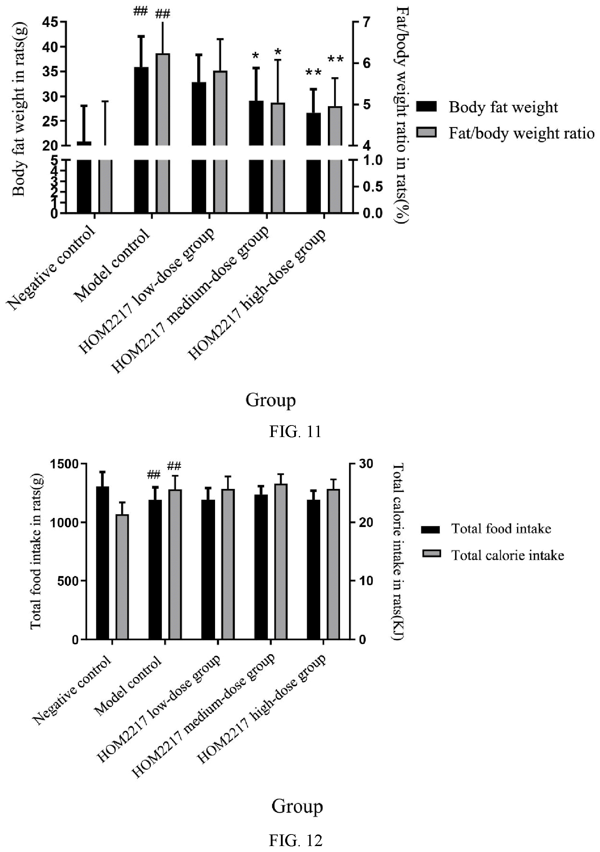

FIG. 11 shows the effect of *L. plantarum* HOM2217 according to the present invention on body fat weight and fat/body ratio of obesity model rats. Note: ## indicates an extremely significant difference from the negative control group ($p < 0.01$); * indicates a significant difference from the model control group ($p < 0.05$), and ** indicates a significant difference from the model control group ($p < 0.01$).

FIG. 12 shows the effect of *L. plantarum* HOM2217 according to the present invention on the total food intake and total caloric intake of obesity model rats. Note: # indicates a significant difference from the negative control group ($p < 0.05$); * indicates a significant difference from the model control group ($p < 0.05$).

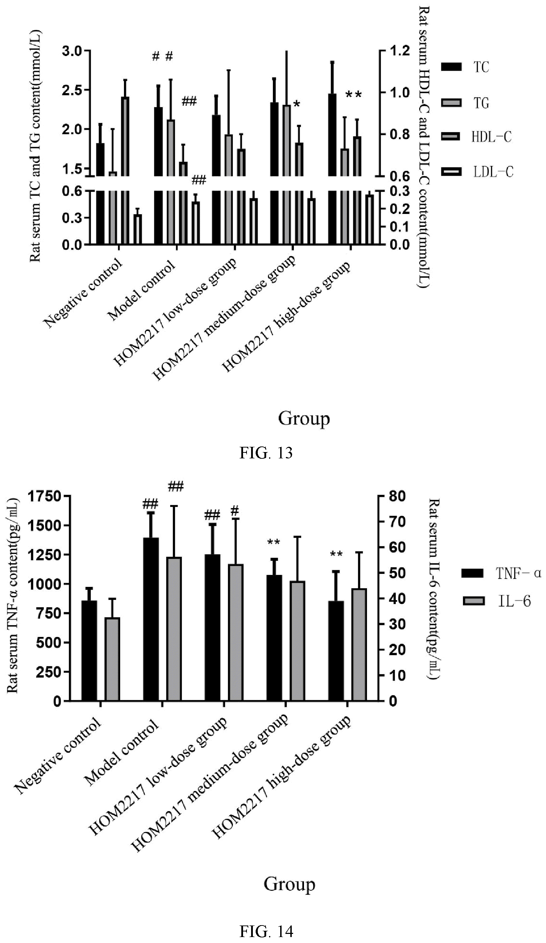

FIG. 13 shows the effect of *L. plantarum* HOM2217 according to the present invention on serum total cholesterol (TC), serum triglyceride (TG), serum high-density lipoprotein cholesterol content (HDL-C) and serum low-density lipoprotein cholesterol content (LDL-C) in obesity model rats. Note: ## indicates an extremely significant difference from the negative control group ($p < 0.01$); * indicates a significant difference from the model control group ($p < 0.05$); ** indicates an extremely significant difference from the model control group ($p < 0.01$).

FIG. 14 shows the effect of *L. plantarum* HOM2217 according to the present invention on serum cytokines TNF- $\alpha$ and IL- 6 in obesity model rats. Note: # indicates an extremely significant difference from the negative control group ($p < 0.05$); ## indicates an extremely significant difference from the negative control group ($p < 0.01$); ** indicates an extremely significant difference from the model control group ($p < 0.01$).

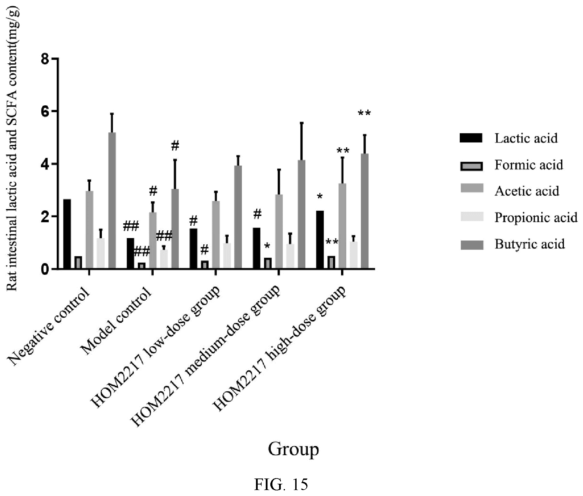

FIG. 15 shows the effect of *L. plantarum* HOM2217 according to the present invention on the content of cecum lactate and short-chain fatty acid (SCFA) in obesity model rats. Note: ## indicates an extremely significant difference from the negative control group ($p < 0.01$); * indicates a significant difference from the model control group ($p < 0.05$); ** indicates an extremely significant difference from the model control group ($p < 0.01$).

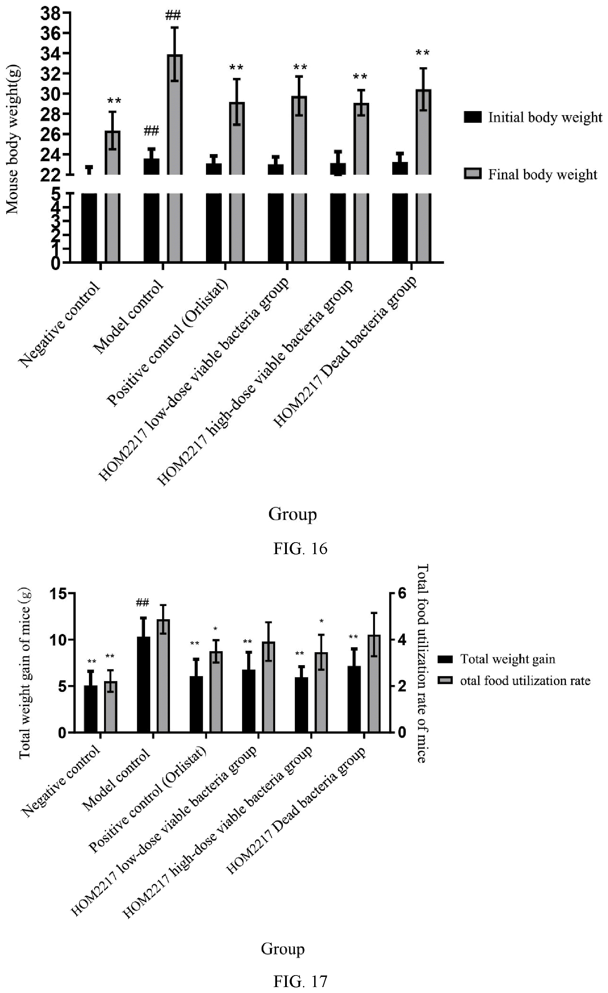

FIG. 16 shows the effect of *L. plantarum* HOM2217 according to the present invention on body weight of obesity model mice. Note: ## indicates a significant difference from the negative control group ($p < 0.01$); ** indicates a significant difference from the model control group ($p < 0.01$).

FIG. 17 shows the effect of *L. plantarum* HOM2217 according to the present invention on the total weight gain and total food utilization rate of obesity model mice. Note: # indicates an extremely significant difference from the negative control group (p < 0.05); ## indicates an extremely significant difference from the negative control group (p < 0.01); ** indicates an extremely significant difference from the model control group (p < 0.01).

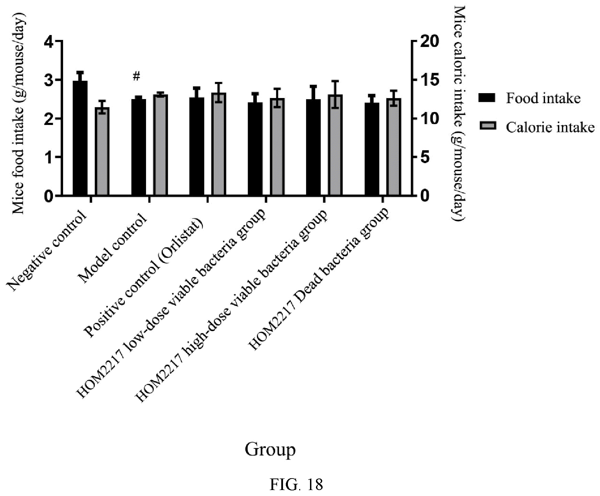

FIG. 18 shows the effect of *L. plantarum* HOM2217 according to the present invention on food intake and caloric intake of obesity model mice. Note: # indicates a significant difference from the negative control group (p < 0.05); * indicates a significant difference from the model control group (p < 0.05).

**Microbiological Collection**

Biological material: HOM2217 strain, classification and naming: *Lactiplantibacillus plantarum* (*L. plantarum*), also known as *Lactobacillus plantarum*, was deposited in the China General Microbiological Culture Collection Center (CGMCC) on September 9, 2022, with deposit number of CGMCC No. 25683; the deposit address is: Institute of Microbiology, Chinese Academy of Sciences, No. 3, Courtyard 1, Beichen West Road, Chaoyang District, Beijing.

**Identification**

The *Lactiplantibacillus plantarum* HOM2217 strain of the present invention, also known as *Lactobacillus plantarum* HOM2217 strain, was sent to the Institute of Microbiology, Chinese Academy of Sciences for identification in May 2023.

The detection and identification conclusions are as follows: Under the conditions of this laboratory, according to the comprehensive analysis of experimental data such as cell morphology, physiological and biochemical characteristics, 16S rRNA gene sequence, *pheS* gene sequence of the test strain, and referring to the "*Bergey's Manual of Systematic Bacteriology*" and relevant research paper of International Journal of Systematic and Evolutionary Microbiology, the identification result of the test strain (strain number: HOM2217) is: *Lactiplantibacillus plantarum.* Synonym: *Lactobacillus plantarum*.

The cell morphology of this strain is: rod-shaped; the physiological and biochemical characteristics are: Gram-positive, catalase negative (-), oxidase negative (-); 16S rRNA gene sequence is shown in SEQ ID NO: 1, and *pheS* gene sequence is shown in SEQ ID NO: 9.

**DETAILED DESCRIPTION**

The present invention discloses strains, characteristics and applications, and those skilled in the art can appropriately improve process parameters with reference to the content herein. It should be particularly noted that all similar substitutions and amendments are obvious to those skilled in the art, and are considered to be included in the present invention. The method and application of the present invention have been described through the preferred embodiments. It is obvious that those skilled in the art can make amendments or appropriate changes and combinations to the method and application described herein without departing from the content, spirit and scope of the present invention to implement and apply the technology of the present invention.

In order to further illustrate the technical means and effects adopted by the present invention to achieve the intended purpose, the technical solution of the present invention will be further described below in conjunction with specific examples, but are not limited thereto. Any amendments or equivalent substitutions to the technical solutions of the present invention, without departing from the spirit and scope of the technical solutions of the present invention, shall fall within the protection scope of the present invention. The experimental methods without specific conditions in the following embodiments are implemented according to conventional methods and conditions in the art.

In order to make the technical problems to be solved of the present invention, technical solutions adopted and advantages clearer, the present invention will be described in detail below with reference to the drawings and specific embodiments. The following examples are intended to illustrate the present invention, but are not intended to limit the scope of the present invention.

It should be noted that the experimental methods used in the present invention are conventional methods unless otherwise specified.

Unless otherwise specified, the reagents and materials used in the present invention are all prepared by conventional methods or obtained commercially.

The *Lactiplantibacillus plantarum* (*L. plantarum*) is a class of gram-positive, non-motile, non-spore forming bacterium. Its cells are straight rod-shaped, usually 0.9 to 1.2 μm × 3.0 to 8.0 μm, single, paired or short chain shaped, bacterial genus, widely distributed in many foods including dairy products, meat, fish, vegetable and other fermentation products, silage, and mucosa of animal and human, such as oral cavity, gastrointestinal tract, vagina, etc. This strain has been listed in the European Union's Qualified Presumption of Safety (QPS) recommended biological agents list, and in the International Dairy Federation's (IDF) "List of Microorganisms with a History of Safe Use in Food", and has received GRAS (Generally Recognized As Safe) designation of the United States Food and Drug Administration. In 2010 years, National Health Commission of the People's Republic of China also approved *L. plantarum* as an edible strain.

The present invention develops a strain with weight loss function, which can reduce the content of cholesterol, inhibit the differentiation of 3T3-L1 preadipocytes; significantly reduce the body weight of obesity model rats, reduce the total weight gain and total food utilization rate of obesity model rats, significantly reduce the body fat weight and fat/body weight ratio of obesity model rats, increase the content of serum high-density lipoprotein cholesterol, effectively increase the content of intestinal short-chain fatty acids, and reduce the expression level of cellular inflammatory factor TNF-$\alpha$ in serum. It can significantly reduce the body weight in obesity model mice, and reduce the total weight gain and total food utilization rate in obesity model rats. The strain can produce lactic acid and short-chain fatty acids with high yield, has good production performance, and can be developed into foods, health foods and drugs related to weight loss.

In the present invention, *"Lactiplantibacillus plantarum"* and *"Lactobacillus plantarum"* are used interchangeably.

The strain of *L. plantarum* HOM2217 used in the present invention was isolated from healthy human breast milk, and the specific isolation method is as described in Example 1.

**Example**

**Example 1. Isolation and Identification of *L. plantarum* HOM2217 Strain**

(1) Preparation of Artificial Gastrointestinal Fluid

Artificial gastric fluid: 16.4 mL of dilute hydrochloric acid (1 mol/L) was taken, 800 mL of water was added, the pH was adjusted to 3.0, and 10g of pepsin was added. The mixture was shaken well, added with water to 1000 mL, centrifuged at 5000 rpm for 5 min, the supernatant was taken, filtered with a 0.22$\mu$m filter membrane for sterilization, and stored at -20°C for later use.

Artificial intestinal fluid: 6.8g of potassium dihydrogen phosphate was taken, 500 mL of water was added, and the pH was adjusted to 6.8 with a 0.4% (0.1 mol/L) sodium hydroxide solution. Separately, 10 g of pancreatin and 3 g of pig bile salt were weighted, an appropriate amount of water was added to dissolve them. The two solutions were mixed, added with water to 1000 mL, centrifuged at 5000 rpm for 5 min. The supernatant was taken, filtered and sterilized with a 0.22 $\mu$m filter membrane, and stored at -20°C for later use.

(2) Preparation of Isolation Medium

MRS liquid medium: MRS broth medium (Cat. No. CM1163, OXOID, UK) was prepared according to the instructions, sterilized at 121°C for 15 min, and stored at 2 to 8°C for one week in the dark.

MRS-Cys solid medium containing bromocresol purple: 0.04g of bromocresol purple was added to each liter of MRS solid medium (Cat. No.: CM1175, OXOID, UK), and stirred well. Sterilization was performed at 121°C for 15 min, about 15 mL of the medium was poured into each petri dish in a clean bench and was allowed to solidify for later use.

(3) Isolation and Screening of *L. plantarum* HOM2217

The isolated breast milk was collected with a sterile aerobic tube and stored at low temperature in an aerobic environment. The experiment began on the day of sampling. About 1g of breast milk sample was weighted and added into an aerobic tube containing 9 mL of MRS liquid medium, aerobically incubated at 37°C for 24 h. After centrifugation at 8000 rpm for 10 min, the supernatant was discarded, and 10 mL of artificial gastric fluid was added. After mixing, the mixture was incubated aerobically at 37°C for 3 h. The sample was diluted by a 10-fold dilution method, diluted to a concentration of $10^{-6}$. 100$\mu$L of each dilution was taken from the original solution to the concentration of $10^{-6}$, and spread onto MRS solid medium plates containing bromocresol purple, aerobically static incubated at 37°C for 48 h.

Single colonies with yellowing edges were picked, streaked, and purified 3-4 times until single colonies were observed. Gram staining and microscopy of colony morphology were performed simultaneously. Single colonies were then transferred to liquid medium for pure culture, preserved with glycerol, and stored at -80°C.

(4) Morphological Observation of *L. plantarum* HOM2217 Strain

*L. plantarum* HOM2217 was aerobic cultured on MRS agar medium at 37°C for 24 h. The colonies were approximately 3 mm in diameter, raised, round, the surface was moist and smooth, and were white, occasionally light yellow or dark yellow. It was observed under an optical microscope that the bacteria were rod-shaped with round-ended. The size of the bacteria was about 0.9 to 1.2 $\mu$m $\times$ 3 to 8 $\mu$m, arranged individually or in pairs, or in short chain, Gram-positive, and did not form spores. At the same time, the other two strains with the same morphology were screened and named as *L. plantarum* S1-6 and S2-13.

(5) Identification of Strain of Plant HOM2217

(5) Identification of *L. plantarum* HOM2217

16S rRNA gene identification of the strain: DNA was extracted from the stored three strains HOM2217, S1-6 and S2-13, respectively, and DNA amplification was performed. PCR amplification and agarose gel electrophoresis were performed using universal primers 27F (SEQ ID NO: 7) and 1492R (SEQ ID NO: 8). Then, the gel was cut, recovered, and sequenced. The isolated strains were sequenced using 16S rDNA. According to its 16S rDNA sequence, it was aligned in the NCBI database using the BLAST tool, and the phylogenetic tree was constructed using Mega 7.0 software, and the results were shown in FIG. 1. As shown in FIG. 1, all three strains were identified as *L. plantarum*, named *L. plantarum* HOM2217, S1-6, and S2-13 strains, respectively. The sequence of the 16S rRNA gene was shown in SEQ ID NO: 1:

TATACATGCAGTCGAACGAACTCTGGTATTGATTGGTGCTTGC

ATCATGATTTACATTTGAGTGAGTGGCGAACTGGTGAGTAACACGTGGGAA

ACCTGCCCAGAAGCGGGGGATAACACCTGGAAACAGATGCTAATACCGCAT

AACAACTTGGACCGCATGGTCCGAGTTTGAAAGATGGCTTCGGCTATCACT

TTTGGATGGTCCCGCGGCGTATTAGCTAGATGGTGGGGTAATGGCTCACCAT

GGCAATGATACGTAGCCGACCTGAGAGGGTAATCGGCCACATTGGGACTGA

GACACGGCCCAAACTCCTACGGGAGGCAGCAGTAGGGAATCTTCCACAAT

GGACGAAAGTCTGATGGAGCAACGCCGCGTGAGTGAAGAAGGGTTTCGGC

TCGTAAAACTCTGTTGTTAAAGAAGAACATATCTGAGAGTAACTGTTCAGG

TATTGACGGTATTTAACCAGAAAGCCACGGCTAACTACGTGCCAGCAGCCG

CGGTAATACGTAGGTGGCAAGCGTTGTCCGGATTTATTGGGCGTAAAGCGA

GCGCAGGCGGTTTTTTAAGTCTGATGTGAAAGCCTTCGGCTCAACCGAAGA

AGTGCATCGGAAACTGGGAAACTTGAGTGCAGAAGAGGACAGTGGAACT

CCATGTGTAGCGGTGAAATGCGTAGATATATGGAAGAACACCAGTGGCGAA

GGCGGCTGTCTGGTCTGTAACTGACGCTGAGGCTCGAAAGTATGGGTAGCA

AACAGGATTAGATACCCTGGTAGTCCATACCGTAAACGATGAATGCTAAGTG

TTGGAGGGTTTCCGCCCTTCAGTGCTGCAGCTAACGCATTAAGCATTCCGC

CTGGGGAGTACGGCCGCAAGGCTGAAACTCAAAGGAATTGACGGGGGCCC

GCACAAGCGGTGGAGCATGTGGTTTAATTCGAAGCTACGCGAAGAACCTTA

CCAGGTCTTGACATACTATGCAAATCTAAGAGATTAGACGTTCCCTTCGGGG

ACATGGATACAGGTGGTGCATGGTTGTCGTCAGCTCGTGTCGTGAGATGTT

GGGTTAAGTCCCGCAACGAGCGCAACCCTTATTATCAGTTGCCAGCATTAA

GTTGGGCACTCTGGTGAGACTGCCGGTGACAAACCGGAGGAAGGTGGGG

ATGACGTCAAATCATCATGCCCCTTATGACCTGGGCTACACACGTGCTACAA

TGGATGGTACAACGAGTTGCGAACTCGCGAGAGTAAGCTAATCTCTTAAAG

CCATTCTCAGTTCGGATTGTAGGCTGCAACTCGCCTACATGAAGTCGGAATC

GCTAGTAATCGCGGATCAGCATGCCGCGGTGAATACGTTCCCGGGCCTTGT

ACACACCGCCCGTCACACCATGAGAGTTTGTAACACCCAAAGTCGGTGGG

GTAACCTTTTAGGAACCAGCCGC

In addition, the *L. plantarum* S1-6 and S2-13 strains isolated simultaneously with the *L. plantarum* HOM2217 of the present invention are used as control strains in the present invention, and the sequences of 16S rRNA genes are shown in SEQ ID NOs: 10 and 11, respectively. By comparing the 16S rRNA gene sequences of the *L. plantarum* HOM2217 strain of the present invention and the *L. plantarum* S1-6 and S2-13 strains used as control strains in the present invention, it can be seen that both the strain S1-6 and the strain S2-13 have the same 1431 bases as the HOM2217 strain of the present invention, and the sequence similarity is 99.38%.

(6) Identification of strain by random amplified polymorphic DNA (RAPD): DNA was extracted from the preserved strain, the strain DNA was used as a template, and a total of 5 primers of OPA-02, OPA-18, OPL-07, OPL-16 and OPM-05 were used for PCR, as shown in Table 1. DNA fragments capable of showing polymorphism were amplified by PCR, different DNA differences were present after gel electrophoresis, and the results were analyzed by cluster analysis software, and the results were shown in FIG. 2. As shown in FIG. 2, the *L. plantarum* HOM2217 strain is unique and different from other *L. plantarum* strains.

Table 1

| Primers | Sequence | SEQ ID NO |
|---------|----------|-----------|
| OPA-02 | 5'- TGC CGA GCT G -3' | 2 |
| OPA-18 | 5'- AGG TGA CCG T -3' | 3 |
| OPL-07 | 5'- AGG CGG GAA C -3' | 4 |
| OPL-16 | 5'- AGG TTG CAG G -3' | 5 |
| OPM-05 | 5'- GGG AAC GTG T -3' | 6 |
| 27F | 5'- AGTTTGATCMTGGCTCAG -3' | 7 |
| 1492R | 5'- GGTTACCTTGTTACGACTT -3' | 8 |

**Example 2. Test on the Ability to Inhibit Common Pathogenic Bacteria**

(1) Indicator Bacteria Activation

The indicator strains *Escherichia coli* ATCC8739; *Staphylococcus aureus* ATCC6538; *Salmonella typhimurium* ATCC14028; *Pseudomonas aeruginosa* ATCC9027; *Listeria monocytogenes* ATCC19111 and *Clostridium difficile* ATCC9689 were all purchased from the China Center of Industrial Culture Collection. Indicator strains (*Escherichia coli* ATCC8739; *Staphylococcus aureus* ATCC6538; *Salmonella typhimurium* ATCC14028; *Pseudomonas aeruginosa* ATCC9027; *Listeria monocytogenes* ATCC19111) were inoculated into TSB medium at an inoculation amount of 1% of the total medium, and cultured aerobically at 37°C and 180 rpm for 24h for later use. *Clostridium difficile* ATCC9689 was inoculated into BHI medium at an inoculation amount of 1% of the total amount of the medium, and cultured anaerobically at 37°C for 24 h for later use.

(2) Activation of *L. plantarum*

The *L. plantarum* HOM2217, S1-6 and S2-13 strains, cryopreserved at - 80°C as prepared in Example 1, were inoculated into the sterilized MRS-Cys liquid medium described in Example 1 at an inoculation amount of 1% of the total medium, respectively, and allowed to stand and cultured anaerobically at 37°C for 24 hours. After two activations, the strain fermentation solution was obtained. Then the strain fermentation solution was centrifuged at 8000 rpm for 10 min, and the supernatant was taken for bacteriostatic test.

(3) Plate Preparation

The sterilized tryptic soy agar (TSA) medium was heated to completely melt, poured into a petri dish, placed on a horizontal platform to form an agar layer with a uniform thickness, and solidified. The indicator strains were added to the TSA medium, shaken evenly, poured into a pre-prepared TSA blank agar plate, and allowed to stand for coagulation.

(4) Bacteriostatic Test

The Oxford cup was gently placed on the plate with sterile forceps, with a certain distance between the holes, and 0.2 mL of the supernatant of the lactic acid bacteria fermentation solution to be tested was respectively added thereto. After the mixture was placed in a refrigerator at 4°C for 24 hours, cultured in an incubator at 37°C for at least 18 hours to observe the occurrence of the inhibition zone. The inhibition zone was formed and then measured with a ruler. The liquid medium (MRS) in Example 1 was used as a negative control, and the *L. plantarum* S1-6 and S2-13 were used as positive control strains. Each sample was tested in triplicate. The antibacterial results were shown in Table 2.

Table 2. Inhibitory Effect of *L. plantarum* HOM2120 Strain on Pathogenic Bacteria

| Sample | *E. coli* | *Salmonella* | *Staphylococcus aureus* | *Pseudomonas aeruginosa* | *Listeria monocytogenes* | *Clostridium difficile* |
|---|---|---|---|---|---|---|
| MRS-Cys (Negative Control) | - | - | - | - | - | - |
| S1-6 | ++ | ++ | + | + | + | + |
| S2-13 | ++ | ++ | + | + | + | + |
| HOM2217 | +++ | +++ | ++ | +++ | +++ | +++ |

Note: "-" no bacteriostatic activity; "+" 11-16 mm; "+ +" 17-22 mm; "+++" ≥ 23 mm

It can be seen from Table 2 that, compared with the positive control S1-6 and S2-13 strains, the *L. plantarum* HOM2217 strain has a strong inhibitory effect on 6 pathogenic bacteria, and has a strong inhibitory effect on *E. coli*, *Salmonella*, *P. aeruginosa*, *L. monocytogenes* and *C. difficile*, indicating that the strain has a good ability to inhibit pathogenic bacteria.

**Example 3. Test on the Ability to Inhibit Obesity Bacteria**

(1) Strain Activation

The indicator bacterium *Enterobacter cloacae* ATCC13047 and the *L. plantarum* HOM2217, S1-6 and S2-13 strains prepared in Example 1 were inoculated into the sterilized MRS liquid medium described in Example 1 at an inoculation amount of 1% of the total medium, incubated for 24 hours at 37°C with aerobic condition. After two activation, fermentation solution of the strain was obtained. The solution was then centrifuged at 8000 rpm for 10 min and collected the bacterial cells. After washing 3 times with sterile physiological saline, the bacterial count was adjusted to $1 \times 10^8$ CFU/mL for later use.

(4) Co-Culture Growth Antagonism Test

The cultured *E. cloacae* ATCC13047 and strain suspensions of *L. plantarum* HOM2217, S1-6 and S2-13 strains were inoculated into the sterilized MRS liquid medium described in Example 1 at an inoculation amount of 1% of the total amount of the medium. The culture was vortexed for 20 s. A culture inoculated only with *E. cloacae* ATCC13047 was used as a positive control, allowed to stand and cultured aerobically at 37°C for 18 hours, and plate viable bacteria counts were performed with MRS medium. Three parallel groups were processed each time, and the data were analyzed by using Graph Pad prism9 software for one-way ANOVA and Dunnett's t-test, i.e., pairwise comparison and analysis of the means between multiple experimental groups and a control group. The results were shown in FIG. 3.

Increasing evidence suggests that intestinal microbiota plays an important role in obesity, insulin resistance and related liver diseases. Recently, *E. cloacae* found in obese mice is considered as a pathogenic bacterium in the intestinal tract and is one of the direct culprits for obesity.

As shown in FIG. 3, compared with the positive control strains S1-6 and S2-13, the *L. plantarum* HOM2217 strain and *E. cloacae* were co-cultured for 18 hours, can significantly ($p < 0.01$) inhibit the growth of *E. cloacae*, having potential value in weight loss.

**Example 4. Test on the Ability to Pass Through Gastrointestinal tract**

(1) Activation of Strains

The *L. plantarum* HOM2217, S1-6 and S2-13 strains prepared in Example 1 were inoculated into the sterilized MRS liquid culture medium described in Example 1 at an inoculation amount of 1% of the total amount of the culture medium, allowed to stand and cultured aerobically at 37°C for 24 hours. After be activated twice, the strain fermentation broth was obtained.

(2) Preparation of Artificial Gastric Fluid

Into 16.4 ml of dilute hydrochloric acid, about 800 ml of water and 10 g of pepsin were added, shook well, the pH was adjusted to 3.0, the water was added to 1000 ml, and filtered with a 0.2 μm microporous membrane for later use.

(3) Preparation of Artificial Intestinal Fluid

6.8 g of potassium dihydrogen phosphate was weighted and dissolved in 500 mL of water, and the pH was adjusted to 6.8. Separately, 10 g of trypsin and 3 g of pig bile salt were weighted, dissolved in an appropriate amount of water. The two solutions were mixed, water was added to 1000 mL, and filtered with a 0.22 um sterile membrane under a sterile environment for later use.

(4) Evaluation of Survival Capacity of Strains in Simulated Gastrointestinal tract

1 mL of activated bacterial solution of the strain to be tested was added to 9 mL of artificial gastric fluid (pH3.0), mixed well, and the number of viable bacteria was counted, and incubated at 37°C for 3 h in an incubator before counting the viable cells again. After culturing in artificial gastric fluid for 3 h, all the bacteria cells were transferred into an equal volume of artificial intestinal fluid (pH6.8), mixed well, and incubated at 37°C. The viable cell counts were performed on MRS medium at 3 h and 24 h respectively, and the survival rate was calculated by the following formula:

Survival rate in the gastric fluid for 3 hours (%) = [logCFUN1/logCFUN0] × 100% Survival rate in the intestinal fluid for 3 hours (%)= [logCFUN2/logCFUN0] × 100% Survival rate in the intestinal fluid for 24 hours (%) = [logCFUN3/logCFUN0] × 100%

N0 = the viable count of *L. plantarum* before treatment, N1 = the viable count of *L. plantarum* after gastric juice treatment for 3 hours, N2 = the viable count of *L. plantarum* after intestinal juice treatment for 3 hours, N3 = the viable count of *L. plantarum* after intestinal juice treatment for 24 hours.

Table 3. The survival rate of *L. plantarum* HOM2217 strain in the simulated gastrointestinal fluids

| Group | Survival rate in gastric fluid for 3 hours | Survival rate in intestinal fluid for 3 hours | Survival rate in intestinal fluid for 24 hours |
|---|---|---|---|
| S1-6 | 99.91±0.37% | 98.50±1.97% | 89.52±1.14%[C] |
| S2-13 | 99.64±0.32% | 98.48±1.33% | 95.23±0.86%[B] |
| HOM2217 | 100.39±0.79% | 99.74±1.15% | 99.95±0.99%[A] |

It can be seen from Table 3 that the survival rate of the *L. plantarum* HOM2217 strain can reach 100% after 3 hours of simulated gastric juice treatment. After further treatment with simulated intestinal fluid for 24 hours, the survival rate of can still reach up to 99% or more, and the survival rate is very significantly ($p < 0.01$) better than the intestinal juice tolerance of the other two strains of *L. plantarum.* It indicates that the *L. plantarum* HOM2217 strain has a high survival rate in the intestinal tract, laying a foundation for its colonization and effective function in the intestinal tract.

## Example 5. Intestinal Epithelial Cells Adhesion Capacity Test

(1) Preparation of Cell Medium

Complete medium: Into high-sugar DMEM medium, 10% of inactivated fetal bovine serum (FBS) and 1% (v/v) of antibiotic (100 U/mL penicillin, 100 $\mu$g/mL streptomycin) were added, mixed and then stored at 4 °C.
Incomplete medium: High-sugar DMEM medium supplemented with 10% inactivated fetal bovine serum (FBS), mixed and stored at 4 °C.

(2) Cell Recovery and Culture

Human colorectal adenocarcinoma Caco-2 cells were purchased from the Cell Resource Center, Institute of Basic Medical Sciences, Chinese Academy of Medical Sciences. The Caco-2 cells were resuspended in a fresh culture solution, uniformly dispersed in a culture flask, cultured at 37°C under a gas condition of 5% $CO_2$ and 95% air. The culture solution was replaced every 48 h during recovery. When the cells grew well (80% confluence), Caco-2 cells were digested with trypsin-EDTA solution at 37°C. The cell concentration was adjusted to $1 \times 10^5$ cells/mL after digestion, and the cells were inoculated in a 24-well plate and cultured until the cells reached 80% confluence.

(3) Activation of *L. plantarum*

The *L. plantarum* HOM2217, S1-6 and S2-13 strains, cryopreserved at - 80 °C as prepared in Example 1, were respectively inoculated into the sterilized MRS liquid medium described in Example 1 at an inoculation amount of 1% of the total amount of the medium, allowed to stand and cultured aerobically at 37°C for 24 hours and activated twice, and then the strain fermentation broth was obtained.

(4) Adhesion Experiment

The bacterial cells grown in the corresponding medium were collected by centrifugation at 8000 rpm for 10 min. After washing the bacterial cells with DPBS for 3 times, the bacterial cells were resuspended in the incomplete medium, and the concentration of the bacterial cells was adjusted to $10^7$ cfu/mL. 1 mL of the above bacterial suspension was added to a 24-well plate containing Caco-2 cells that have grown to a single layer, and incubated at 37°C for 2 h in a 5% $CO_2$ incubator. After incubation, the cells were washed with sterile Dulbecco's phosphate buffered saline (DPBS) for 3 times. The Caco-2 cells were digested with trypsin-EDTA solution at a temperature of 37°C, the number of cells and the number of viable bacteria before and after adhesion were counted. Each sample was tested in triplicate, and the results were shown in Table 4.

Table 4. Adhesion Capacity of *L. plantarum* HOM2217 Strain to Caco-2 cells

| Strain Name | Adhesion Rate (%) | Adhesion Index (CFU/Cell) |
|---|---|---|
| S1-6 | 0.98±0.15%[B] | 0.58±0.09[B] |
| S2-13 | 1.31±0.07%[B] | 0.79±0.21[B] |
| HOM2217 | 4.32±0.46%[A] | 4.91±0.53[A] |

Note: In the same column, different capital letters for the superscript indicate highly significant differences (p < 0.01).

Adhesion index = number of bacteria after adhesion/number of cells per plate

Adhesion rate = number of bacteria after adhesion/number of bacteria before adhesion

The results in Table 4 show that the adhesion rate of *L. plantarum* HOM2217 strain to human colon cancer cells Caco-2 is 4.32%, the adhesion index is 4.91, and compared with the other two strains of *L. plantarum* S1-6 and S2-13, it can significantly (p < 0.01) improve the adhesion ability of small intestinal epithelial cells.

**Example 6. Test for Production Capacity of Lactic Acid and Short Chain Fatty Acid**

The *L. plantarum* HOM2217, S1-6 and S2-13 strains, cryopreserved at - 80 °C as prepared in Example 1, were respectively inoculated into the sterilized MRS liquid medium described in Example 1 at an inoculation amount of 1% of the total amount of the medium, allowed to stand and cultured aerobically at 37°C for 24 hours. After being activated twice, the strain fermentation solution was obtained. Then the solution was centrifuged at 8000 rpm for 10 min, and the supernatant was taken and the contents of lactic acid and short-chain fatty acids were detected by gas chromatography. The MRS liquid medium described in Example 1 was used as a negative control. Each sample was performed in triplicate. The data were analyzed by SPSS25.0 software for one-way ANOVA analysis and Duncan's multiple comparison.

Table 5. Lactic acid and short-chain fatty acid production capacity of the *L. plantarum* HOM2217 strain

| Sample | Lactic Acid (g/L) | Acetic Acid (g/L) | Formic Acid (g/L) | Propionic Acid (mg/L) | Butyric Acid (mg/L) | Isobutyric Acid (mg/L) |
|---|---|---|---|---|---|---|
| MRS-Cys (Negative Control) | 0.54±0.01[C] | 2.13±0.01[C] | 0.16±0.00[C] | 11.4±0.2[C] | 12.3±0.3[B] | 10.0±0.2[C] |
| S1-6 | 21.75±0.17[C] | 2.60±0.06[B] | 0.45±0.01[B] | 14.5±0.3[B] | 12.7±0.3[B] | 10.4±0.2[BC] |
| S2-13 | 23.44±0.17[B] | 2.59±0.01[B] | 0.45±0.01[B] | 15.0±0.4[B] | 12.5±0.3[B] | 10.5±0.1[B] |
| HOM2217 | 25.39±0.22[A] | 2.73±0.03[A] | 0.56±0.02[A] | 19.9±0.2[A] | 14.3±0.3[A] | 10.7±0.2[A] |

Note: In the same column, different capital letters for the superscript indicate highly significant differences (p < 0.01).

As can be seen from Table 5, compared with the other two strains *L. plantarum* S1-6 and S2-13 strain, the *L. plantarum* HOM2217 strain can significantly (p < 0.01) increase the productivity of lactic acid and short-chain fatty acids (formic acid, acetic acid, propionic acid, butyric acid and isobutyric acid).

**Example 7. Antibiotic Sensitivity Test**

The drug susceptibility test was carried out according to ISO10932-2010, "Milk and milk products - Determination of the minimal inhibitory concentration (MIC) of antibiotics applicable to bifidobacteria and non-enterococcal lactic acid bacteria (LAB)", established by the International Organization for Standardization.

(1) Preparation of Medium

MRS liquid medium: preparation method is the same as in Example 1.

LSM liquid medium: 21.06g of Iso-Sensitest medium (Cat. No. CM0473B, OXOID, UK) was weighed, 5.2 g of MRS broth was weighed, added with water to 0.5 L, the pH was adjusted to 6.85 ± 0.1, and sterilized at 121°C for 15 min, and the pH should be 6.7 ± 0.1 for later use, and stored at 2 - 8°C for one week in the dark.

Activation and Proliferation of *L. plantarum*

The *L. plantarum* HOM2217 strain prepared in Example 1 was inoculated into the sterilized liquid medium (MRS) described in Example 1 at an inoculation amount of 1% of the total amount of the medium, allowed to stand and cultured aerobically at 28°C for 24 hours. After being activated twice, the strain fermentation solution was obtained. The activated *L. plantarum* HOM2217 strain was propagated on MRS agar medium, and allowed to stand and cultured aerobically at 28°C for 48 hours.

(2) Preparation of Antibiotic Microdilution Plate

0.0512g of antibiotic was weighed, into which 10 mL of solvent was added, wherein chloramphenicol and erythromycin were dissolved in ethanol (without filtration), ampicillin was dissolved in phosphate buffer (pH 8.0, 0.1 mol/L), and other antibiotics were dissolved in water. After shaking to dissolve, the solution was filtered with a 0.22μm filter membrane, sub-packaged into EP tubes, with a concentration of 5120 μg/mL (5.12 mg/mL), and stored at -20°C for later use. The antibiotic stock solution was diluted with water (ampicillin with phosphate buffer) to a suitable concentration range. 50μL of diluent was added to the wells of the microdilution plate.

(3) Preparation of Bacterial Suspensions

Individual colonies were picked from agar plates. The obtained colonies were suspended in sterile culture tubes containing 2 mL to 5 mL of sterile saline. The obtained colonies were suspended in pre-reduced LSM liquid medium. Colonies were suspended until the solution turbidity reached McFarland Standard 1 or the optical density at 625 nm was 0.16 - 0.2 using the spectrophotometer. The suspension was approximately equivalent to $3 \times 10^8$ CFU/mL. The bacterial suspension was diluted with the recommended medium and the bacterial suspension was diluted 500-fold with MRS liquid medium, as the antibiotic solution will dilute the medium by two times. The diluted bacterial suspension was dispensed within 30 minutes after the preparation. When the freeze plate is used, the frozen antibiotic solution was thawed under aerobic conditions immediately prior to use. 50μL of the diluted suspension was dispensed into each well of the microdilution plate (about $3 \times 10^4$ CFU/well). The plates were incubated at 37°C under static aerobic conditions for 48 h. When an aerobic tank is used, the plates are capped between each plate to create a uniform environment in the aerobic tank. Each experiment was repeated three times, with both positive control group and a negative control group were set. The positive control wells did not contain antibiotics, but included the test strains and medium containing solvents to dissolve the highest concentrations of antibiotics. Negative control wells did not contain the test strain and antibiotics, but included medium.

(4) Reading MIC Results

After 48 hours of incubation, the MIC was visually read. After incubation, the negative control wells were checked for visible bacterial growth. If contamination was found, all data generated by the relevant strain was rejected. Note: If there was no growth in the positive control well, it indicated that the tested strain was sensitive to the solvent used to dissolve the antibiotic. In this case, reading the MIC for that particular antibiotic was meaningless. If the negative and positive controls were normal, the growth of bacteria in each antibiotic was determined visually by comparing them with the positive control. Preferably, the microdilution plate was placed on the top of the frame of the magnifying glass, and the table lamp providing indirect light for easy reading. The growth of bacteria was easily detected under the magnifying glass as sediment at the bottom of the well. Any series of wells in which growth discontinuities were observed were discarded (e.g., grown at 16 μg/mL and 64 μg/mL, but not at 32μg/mL). The endpoint was defined as the lowest antibiotic concentration at which no growth was visually observed. The concentration is the MIC of that antibiotic for that specific strain. Each sample was tested in triplicate, and *Lactobacillus plantarum* ATCC14917 (model strain, designated strain for MIC quality control) was used as a positive control strain. The antibiotic sensitivity results of the *L. plantarum* HOM2217 strain are shown in Table 6. Antibiotic resistance standards for bacterial strains used in food established by the European Food Safety Authority (EFSA) in 2012.

Table 6. Antibiotic susceptibility results of *L. plantarum* HOM2217 strain

| Antibiotics | Judgment Criteria | | *L. plantarum* ATCC14917 (Control Strain) | | *L. plantarum* HOM2217 | |
| --- | --- | --- | --- | --- | --- | --- |
| | Sensitivit y (S) | Resistance (R) | MIC Value | Results | MIC Value | Results |
| Erythromycin | ≦1 | >1 | 0.5±0 | S | 1±0 | S |

(continued)

| Antibiotics | Judgment Criteria | | L. plantarum ATCC14917 (Control Strain) | | L. plantarum HOM2217 | |
|---|---|---|---|---|---|---|
| | Sensitivit y (S) | Resistance (R) | MIC Value | Results | MIC Value | Results |
| Chloramphenicol | ≦8 | >8 | 1±0 | S | 0.83±0.29 | S |
| Tetracycline | ≦32 | >32 | 8±0 | S | 8±0 | S |
| Ampicillin | ≦2 | >2 | 0.5±0 | S | 0.5±0 | S |
| Clindamycin | ≦2 | >2 | 0.5±0 | S | 0.5±0 | S |
| Gentamicin | ≦16 | >16 | 5.3±2.3 | S | 4±0 | S |
| Kanamycin | ≦64 | >64 | 128±0 | R | 128±0 | R |

It can be seen from the results in Table 6 that the *L. plantarum* HOM2217 strain is consistent with the control strain ATCC14917, and is sensitive to erythromycin, chloramphenicol, tetracycline, ampicillin, clindamycin and gentamicin. Therefore, the *L. plantarum* HOM2217 strain of the present invention is relatively safe.

## Example 8. In Vitro Cholesterol Degradation Test

MRS high cholesterol medium: Bile salt (final mass concentration of 3 mg/mL) and cholesterol (final mass concentration of 120μg/mL) were added to the MRS liquid medium described in Example 1.

The *L. plantarum* HOM2217, S1-6 and S2-13 strains prepared in Example 1 were inoculated into the sterilized MRS liquid culture medium described in Example 1 at an inoculation amount of 1% of the total amount of the culture medium, allowed to stand and cultured aerobically at 37°C for 24 hours. After being continuously activated for two generations, the strain was inoculated into the MRS high cholesterol liquid medium, and subjected to anaerobic culture at 37°C for 24 hours, and then centrifuged at 4°C and 8000 rpm for 10 min to collect the supernatant liquid. According to the national standard GB 5009.128-2016 "National Food Safety Standard - Determination of Cholesterol in Food", the cholesterol was determined by gas chromatography. Each sample was performed in triplicate. The cholesterol degradation rate was calculated according to the following formula. Each treatment was tested in triplicate. The data were analyzed by t-test using Graph Pad prism9 software.

$$\text{Cholesterol Degradation Rate (\%)} = \frac{M1\text{-}M2}{M1} \times 100\%$$

where M1 and M2 are the mass (μg) of cholesterol in the supernatant before and after fermentation, respectively.

The results were shown in FIG. 4. As shown in FIG. 4, in the medium containing bile salt, the cholesterol degradation rate of the *L. plantarum* HOM2217 strain was 50.6%, the cholesterol degradation ability was strong and the difference was extremely significant ($p < 0.01$), compared with the other two strains *L. plantarum* S1-6 and S2-13.

## Example 9. In Vitro Test for Inhibition of Lipid Synthesis

(1) Preparation of Cell Medium

Complete medium 1: Into high-sugar DMEM medium, 10% of inactivated new bovine serum (NBS) and 1% (v/v) of antibiotic (100 U/mL penicillin, 100 μg/mL streptomycin) were added, mixed and then stored at 4 °C.

Complete medium 2: High-sugar DMEM medium supplemented with 10% inactivated fetal bovine serum (FBS), mixed and stored at 4 °C.

Cell differentiation medium 1: 3-isobutyl 1-methylxanthine (IBMX) was added to complete medium 2 to achieve a final concentration of 0.5 mmol/L, dexamethasone (DEX) to achieve a final concentration of 1μmol/L, and insulin to achieve a final concentration of 10μg/mL.

Cell differentiation medium 2: Insulin was added to complete medium 2 to achieve a final concentration of 10μg/mL, and stored at 4°C for later use.

(2) Preparation of Test Cell Samples

The *L. plantarum* HOM2217, S1-6 and S2-13 strains prepared in Example 1 were inoculated into sterilized MRS liquid medium described in Example 1 at an inoculum amount of 1% of the total culture medium, allowed to stand and cultured aerobically at 37°C for 24 hours. After being activated twice, the bacterial cells were collected by centrifugation at 8000 r/min for 10 min, washed twice with PBS, and resuspended in PBS solution (0.1 mol/L, pH 7.2 to 7.4) to form a bacterial suspension.

Heat-killed strain (HKS): the bacterial suspension was placed in a water bath, treated at 70°C for 30 minutes to obtain heat-killed bacteria, and stored at - 80°C for later use.

(3) Culture of 3T3-L1 preadipocytes

3T3-L1 preadipocytes (purchased from Institute of Basic Medical Sciences, Chinese Academy of Medical Sciences) were inoculated into complete medium 1 at a ratio of 1:3 for passage, and 3-10 passages of cells were selected for testing.

(4) Cell Viability Assay

The cell count was adjusted to $2 \times 10^5$ cells/mL with complete medium 2. 0.1 mL of cell solution was pipetted into each well and seeded into a 96-well culture plate, cultured for 2 days for later use. After culturing, the 96-well plate cells were taken, the cell culture medium was aspirated, 0.1 mL of different concentrations of test cells (adjusted with complete medium 2) were added to each well, cultured in a 5% $CO_2$ incubator at 37°C for 24 h. The cell viability was detected using the MTS cell proliferation and cytotoxicity assay kit manufactured by Promega. The simplified procedure was as follows: 20$\mu$L of MTS was added to each well, incubated in an incubator in the dark for 1h, and the absorbance was read at 490 nm using a microplate reader. Each treatment used cell culture medium 2 + test cells + MTS as blank control. Complete medium 2 + cells + MTS was used as negative control. The test results are shown in FIG. 5.

It can be seen from the results in FIG. 5 that when the bacterial concentration is not greater than $1 \times 10^9$ TFU/mL, the viability of 3T3-L1 preadipocytes is higher than 100%. Therefore, $1 \times 10^9$ TFU/mL, $1 \times 10^8$ TFU/mL and $1 \times 10^7$ TFU/mL were selected for the evaluation of the lipid lowering effect of cells.

(5) 3T3-L1 Preadipocytes Differentiation

The cultured cells were inoculated with $2 \times 10^5$ cells/mL of complete medium, 1 mL per well into a 24-well culture plate coated with 0.1% gelatin. 3T3-L1 preadipocytes were allowed to confluence until 100% confluence. Three days after confluence was recorded as day 0. Cell differentiation medium 1 was added, and the cells were cultured for 4 days, recorded as day 4. Then, cell differentiation medium 2 was replaced for induction, and the cells were cultured for 2 days, recorded as day 6. After induction differentiation, the cells were cultured in complete medium 2 for 2 days, recorded as day 8. On day 8, 3T3-L1 preadipocytes were differentiated into round mature adipocytes with round triglyceride particles inside, during which the medium was changed every 2 days. The bacterial samples were added at day 0, bacterial samples with different concentrations showing cell viability higher than 100% were added, and co-cultured with 3T3-L1 cells from day 0 to day 8 of induced differentiation.

(6) Oil Red O Staining

The differentiated mature adipocytes were gently washed 3 times with an appropriate amount of PBS, fixed with0.5 mL of 4% paraformaldehyde for 30 min, and then gently washed 3 times with ultrapure water. 0.5 mL of Oil Red O working solution was added to each well, and stained at 37°C for 30 min in the dark. The staining solution was discarded, and then gently washed 3 times with ultrapure water for 3 times. The culture plate was placed under an inverted microscope for observation, and photographed. After the photographing, an equal amount of 1 mL of 100% isopropanol was added to each well to extract Oil Red O staining solution from the lipid droplets, placed at room temperature for 20 min. The extracted staining solution was pipetted and transferred to a clean 96-well culture plate. The absorbance value was measured at 500 nm. Absorbance values were determined at 500 nm, using 100% isopropanol as a blank. The relative lipid content was calculated as follows: relative lipid content (%) = $(OD_{sample})/(OD_{blank}) \times 100\%$. Each treatment was performed in triplicate.The data were analyzed by using Graph Pad prism9 software for one-way ANOVA and Dunnett's t test, i.e., pairwise comparison and analysis of the means between multiple experimental groups and a control group. The results of oil staining and lipid content are shown in FIGS. 6 and 7.

3T3-L1 cells aggregated a large number of lipid droplets in the cells after differentiation and maturation. Oil Red O staining can visually reflect the amount of lipid accumulation after adipogenesis. It can be seen from FIG. 6 that 90% of the cells in the model group were differentiated into mature adipocytes on the day 8 of induction, which showed that the cells were further enlarged, round in shape, and filled with red lipid droplets densely clustered around the nucleus, forming a "ring-like" structure in the form of typical mature fat. Compared with the model control group, after 8 days of induction differentiation using heat-killed *L. plantarum* HOM2217 strain, all three concentrations (low, medium, and high) can significantly reduce the number of intracellular lipid droplets. The quantitative results of Oil Red O dye of adipocytes (FIG. 7) showed that the heat-killed bacteria of the *L. plantarum* HOM2217 strain can significantly ($p < 0.01$) reduce the intracellular lipid content, and have a dose-dependent effect. With the increase of the concentration of heat-killed *L. plantarum* HOM2217 strain, the ability to inhibit differentiation of 3T3-L1 preadipocytes was enhanced, the intracellular lipid content was reduced, and the lipid reduction effect was significantly better than that of the other two strains *L. plantarum* S1-6 and S2-13.

(6) Determination of Intracellular Triglyceride (TG) Content in 3T3-L1

The intracellular triglyceride content was determined according to the instructions of the triglyceride (TG) assay kit produced by Nanjing Jiancheng Bioengineering Institute. The simplified steps were as follows: 2.5μL of sample and 250μL of working solution were added to each well, mixed well, incubated at 37°C for 10 minutes, and the absorbance was read at 510 nm using a microplate reader. Protein concentrations were determined using a total protein quantification kit (BCA method). The simplified steps were as follows: 10μL of sample and 250μL of working solution were added to each well, mixed well, incubated at 37°C for 10 minutes, and the absorbance was read at 562 nm using a microplate reader. The glyceride content per gram of protein was calculated according to the formula.

Calculation formula:

$$\text{Calculation formula: Triglycerides (mmol/gprot)} = \frac{\text{Sample OD value} - \text{Blank OD value}}{\text{Standard OD value} - \text{Blank OD value}} \times \text{Calibrator concentration (mmol/L)} \div \text{Protein concentration of sample to be tested (gprot/L)}$$

Each treatment was performed in triplicate. The data were analyzed by using Graph Pad prism9 software for one-way ANOVA and Dunnett's t test, i.e., pairwise comparison and analysis of the means between multiple experimental groups and a control group. The intracellular triglyceride (TG) content of 3T3-L1 cells was shown in Figure 8. It can be seen from FIG. 8 that the high, medium and low concentrations of heat-killed bacteria of the *L. plantarum* HOM2217 strain significantly ($p < 0.01$) reduce the cellular triglyceride content, and have a dose-dependent effect; with the increase of the concentration of heat-killed bacteria of *L. plantarum* HOM2217, the cellular triglyceride content is reduced, and the lipid reduction effect is significantly better than that of the other two strains *L. plantarum* S1-6 and S2-13.

**Example 10. Preparation Process 1 of Active Bacterial Powder of *L. plantarum* HOM2217**

(1) Culture of Strain

The *L. plantarum strain* HOM2217, cryopreserved at - 80°C prepared in Example 1, was inoculated into a sterilized MRS liquid medium at an inoculation amount of 1%, and cultured at 37°C for 16-24 hours. After two such subcultures, activated seed culture solution was obtained. The seed culture solution was inoculated into the fermentation medium M321 at an inoculation amount of 3%, and cultured aerobically at 35°C of constant temperature . During the fermentation process, a sodium hydroxide solution was automatically added to maintain the pH constant at 5.0, so that the fermentation was stopped until the acid production was stopped, and the fermentation was stopped when the sodium hydroxide solution was no longer added, so as to obtain a high-density culture solution of *L. plantarum* HOM2217 with a viable count of up to 286 billion CFU/mL. Table 7 showed the formulation of fermentation medium M321.

Table 7. The formulation of the fermentation medium M321

| Medium Components | Content (g/L) |
|---|---|
| Glucose | 60 |
| Yeast Extract | 60 |

(continued)

| Medium Components | Content (g/L) |
| --- | --- |
| Magnesium Sulfate | 0.4 |
| Manganese Sulfate | 0.2 |
| Dipotassium hydrogen phosphate | 2 |
| Potassium Dihydrogen Phosphate | 2 |
| Tween-80 | 2 |
| L-Cysteine Salt | 2 |

(2) Preparation of Lyoprotectant

A protectant containing 100 g/L peptone, 20 g/L sucrose, 10 g/L vitamin C and 1 g/L glycerol was prepared by mixing sterile water with the protectant raw materials.

(3) Freeze-Drying

The fermentation broth of the *L. plantarum* HOM2217, after culture, was centrifuged at 6000 rpm for 10 min at 2-8°C. The supernatant was discarded, and the bacterial sludge was collected. The bacterial sludge was washed with 0.9% sterile physiological saline for 1-2 times. The washed bacterial sludge was mixed with the above-mentioned protectant, to achieve a bacterial concentration of $10^{11}$ CFU/mL or more in the mixed bacterial solution. It was then freeze-dried in a freeze dryer, prefrozen at -55°C for 5 hours, vacuumized for 0.05 mbar, the temperature was raised to - 30 °C and dried for 20 hours in a primary drying; then the temperature was raised to - 15 °C, and dried for 15 hours in a primary drying. After the temperature was raised to 35°C, the mixture was dried for 5 hours in a secondary drying. After freeze-drying, the bacterial cake was pulverized by a fine grinder to obtain the freeze-dried bacterial powder. The viable count of the freeze-dried bacterial powder reached $6.57 \times 10^{11}$ CFU/g.

**Example 11. Preparation Process 2 of Active Bacterial Powder of *L. plantarum* HOM2217**

(1) Culture of Strain

The *L. plantarum* HOM2217 strain, cryopreserved at -80 °C prepared in Example 1, was inoculated into a sterilized MRS liquid medium at an inoculation amount of 3%, and cultured at 37°C for 16-24 hours. After two such subcultures, activated seed culture solution was obtained. The seed culture solution was inoculated into the fermentation medium M327 at an inoculation amount of 3%, and cultured aerobically at 35°C of constant temperature. During the fermentation process, a sodium hydroxide solution was automatically added to maintain the pH constant at 6.0, so that the fermentation was stopped until the acid production was stopped, and the fermentation was stopped when the sodium hydroxide solution was no longer added, so as to obtain a high-density culture solution of the *L. plantarum* HOM2217 strain, with a viable count of up to 336 billion CFU/mL. Table 8 showed the formulation of fermentation medium M327.

Table 8. The formulation of the fermentation medium M327

| Medium Components | Content (g/L) |
| --- | --- |
| Glucose | 100 |
| Yeast Extract | 80 |
| Magnesium Sulfate | 0.1 |
| Manganese Sulfate | 0.1 |
| Dipotassium hydrogen phosphate | 4 |
| Potassium Dihydrogen Phosphate | 4 |
| Tween-80 | 1 |
| L-Cysteine Salt | 0.5 |

(2) Preparation of Lyoprotectant

A protectant containing 50 g/L peptone, 60 g/L sucrose, 5 g/L vitamin C and 2 g/L glycerol was prepared by mixing sterile water with the protectant raw materials.

(3) Freeze-Drying

The fermentation broth of the *L. plantarum* HOM2217, after culture, was centrifuged at 6500 rpm for 15 min at 2-8°C. The supernatant was discarded, and the bacterial sludge was collected. The bacterial sludge was washed with 0.9% sterile physiological saline for 1-2 times. The washed bacterial sludge was mixed with the above-mentioned protectant, to achieve a bacterial concentration of $10^{11}$ CFU/mL or more in the mixed bacterial solution. It was then freeze-dried in a freeze dryer, prefrozen at -50°C for 8 hours, vacuumized for 0.03 mbar, the temperature was raised to -25 °C and dried for 15 hours in a primary drying; then the temperature was raised to -10 °C, and dried for 10 hours in a primary drying. After the temperature was raised to 32°C, the mixture was dried for 8 hours in a secondary drying. After freeze-drying, the bacterial cake was pulverized by a fine grinder to obtain the freeze-dried bacterial powder. The viable count of the freeze-dried bacterial powder reached $8.18 \times 10^{11}$ CFU/g.

**Example 12. Preparation Process of Dead Bacterial Powder of *L. plantarum* HOM2217 Strain**

(1) Culture of Strain

The strains cultured in Example 11 were used.

(2) Preparation of Bacterial Powder

The fermentation broth of *L. plantarum* HOM2217 strain obtained by high-density fermentation was treated at 80°C for 30 minutes, 20g of maltodextrin excipient was added per liter of the fermentation broth, and dried by a spray dryer. The number of dead bacteria in the dead bacterial powder was $1.23 \times 10^{12}$ CFU/g when counted under a microscope using a hemocytometer.

**Example 13. In Vivo Test 1 of *L. plantarum* HOM2217 Strain in Obesity Animal Model**

(1) Experimental Animals and Feed

70 healthy SPF-grade male SD rats bred by Beijing Huafukang Biotechnology Co., Ltd were used as experimental animals.
The feed was selected from high-fat feed D12492(60% fat content) and ordinary feed D12450B (10% fat content) produced by Beijing Huafukang Biotechnology Co., Ltd.

(2) Animal Model and Experimental Treatment

Animals were grouped after 1 week of acclimatization. After the end of the acclimatization, 70 rats were randomly divided into 2 groups according to body weight, wherein 10 rats were given maintenance feed as a negative control group, and 60 rats were given high-calorie model feed. After 2 weeks of feeding, the rats given with high-calorie model feed were ranked according to weight gain. 1/3 obesityresistant rats with lower weight gain were eliminated, and 40 obesity-sensitive rats were selected. They were randomly divided into 4 groups according to their weight, which were respectively a model control group and three dosage groups, with 10 rats in each group. The experimental group was divided into three dose groups: a low-dose group ($2.5 \times 10^9$ CFU/Kg BW), a medium-dose group ($1.25 \times 10^{10}$ CFU/Kg BW) and a high-dose group ($2.5 \times 10^{10}$ CFU/Kg BW) by using the bacterial powder of *L. plantarum* HOM2217 prepared in Example 10. Starting from the experiment, each animal in the model control group and each dosage group was given the same amount of high-calorie model feed daily, while the negative control group was given the same amount of maintenance feed in the same manner. Different dosage group was given different doses of the test substance, while the negative control group and the model control group were given sterile water. The test period was 7 weeks.

(3) Detection Indicators and Methods

A. Appearance Indicators

During the experiment, the feeding amount and the remaining feeding amount of each cage of animals were recorded weekly. The feeding amount and total calorie intake (food intake $\times$ calories per kilogram of feed) was calculated. The body weight of rats was recorded weekly, the weight gain and the feed utilization rate was calculated.

$$\text{Feed utilization rate} = \text{weight gain} / \text{food intake} \times 100\%$$

B. Serum Four-Item Assays

At the end of the test, after being fasted for 12 hours, whole blood was obtained by ocular sampling. After standing at room temperature for 2 hours, it was centrifuged at 4000 r/min for 10 min at 4°C to separate the serum. The serum was taken, and the contents of serum total cholesterol, triglyceride, high-density lipoprotein cholesterol and low-density lipoprotein cholesterol of rats in each group were measured by a fully automatic biochemical analyzer.

C. Cytokine Assay

At the end of the experiment, after being fasted for 12 hours, the whole blood was obtained by ocular sampling. After standing at room temperature for 2 hours, it was centrifuged at 4000 r/min for 10 min at 4°C to separate the serum. The serum was taken, and the content of serum cytokines TNF-$\alpha$ and IL-6 was determined by enzyme-linked immunosorbent assay (ELISA) according to the instructions of the kit.

D. Determination of Fat Content

After taking blood from the eyes of rats, the rat was euthanized by cervical dislocation. Body fat (from the testes and perirenal fat pads) was collected and weighed, and the fat/body ratio was calculated.

E. Determination of Lactic Acid and Short-Chain Fatty Acid in Cecal Contents

After taking blood from the eyes of rats, the rats were euthanized by cervical dislocation. Cecal contents were collected, and the content of lactic acid and short-chain fatty acids in the cecal contents were detected by gas chromatography.

(4) Data Processing

Statistical analysis was performed using SPSS software (ANOVA) and the Dennett method for pairwise comparisons of means between multiple experimental groups and a control group. The weight of rats in the negative control group and the model control group before and after the establishment of the obesity model was compared using t-test of independent samples.

(5) Experimental Results

The experimental results were shown in FIGS. 9-15.
It can be seen from the results of FIG. 9 that after the high-calorie model feed was given for 14 days, the weight of the rats increased and there was a significant difference between the model control group and the negative control group, indicating that the model was successfully established. There was no significant difference (p > 0.05) in body weight between each dosage group and the model control group before administration of the test substance. After 49 days of oral administration of the test substance, compared with the negative control group, the weight of rats in the model control group increased, and there was a significant difference (p < 0.05). Compared with the model control group, the final body weight of rats in the high-dose group of *L. plantarum* HOM2217 decreased, with a significant difference (p < 0.05).
It can be seen from the results in FIG. 10 that after the high-calorie model feed was given, the total weight gain and the total food utilization rate of rats in the model control group were both increased compared with the negative control group, and there was a significant difference (p < 0.05). After 49 days of oral administration of the test substance, compared with the model control group, the high-dose group of *L. plantarum* HOM2217 significantly (p < 0.01) reduced the total weight gain and total food utilization rate of rats.
It can be seen from the results in FIG. 11 that after the high-calorie model feed was given, compared with the negative control group, the model control group showed a significant increase in both the body fat weight and fat/body weight ratio of rats. After 49 days of oral administration of the test substance, compared with the model control group, the medium-dose of *L. plantarum* HOM2217 was significantly (p < 0.05) reduced, and the high-dose of *L. plantarum* HOM2217 was extremely significantly (p < 0.01) reduced the body fat weight and fat/body weight ratio in rats.
It can be seen from the results in FIG. 12 that after the high-calorie feed is given, compared with the negative control group, the model control group showed a significant (p < 0.05) decreased in the total food intake of the rats; the total calorie intake of the rats increased significantly (p < 0.05). After 49 days of oral administration of the test substance, there was no significant difference in total food intake and the total caloric intake of rats between the various dosage groups and the model control group (p > 0.05).

It can be seen from the results in FIG. 13 that after oral administration of the test substance to obesity model rats for 49 days, compared with the negative control group, the model control group showed significantly (p < 0.05) increased serum total cholesterol and serum triglyceride, decreased serum high-density lipoprotein cholesterol, and increased serum low-density lipoprotein cholesterol. Compared with the model control group, there was no significant difference (p > 0.05) in serum total cholesterol, serum triglyceride and serum low-density lipoprotein cholesterol in each dose group. The medium-dose of *L. plantarum* HOM2217 significantly (p < 0.05) increased, and the high-dose of *L. plantarum* HOM2217 significantly (p < 0.01) increased the serum high-density lipoprotein cholesterol content of rats.

It can be seen from the results in FIG. 14 that after oral administration of the test substance to obesity model rats for 49 days, compared with the negative control group, the model control group showed increased serum TNF-$\alpha$ and IL-6, and there was an extremely significant difference. Compared with the model control group, the serum IL-6 content in each dosage group had a decreasing trend, but there was no significant difference (p > 0.05). The medium-dose and the high-dose in the *L. plantarum* HOM2217 significantly (p < 0.01) reduced the content of TNF-$\alpha$. Obesity was recognized as a chronic low inflammation state. Inflammation played an important role in abnormal hypertrophy of adipocytes and the increase in endoplasmic reticulum pressure in adipocyte cytoplasm. Macrophages in adipose tissue were considered as an important source of inflammation, which can secrete inflammatory factors (tumor necrosis factor TNF-$\alpha$). TNF-$\alpha$ expression was significantly enhanced in adipocyte in obese animal models. When visceral fat accumulates, the TNF-$\alpha$ released by adipose tissue increases significantly. In obese patients with insulin against (IR) and obese patients with type 2 diabetes, the TNF-$\alpha$ expression of adipose tissue and muscle tissue was significantly increased. Therefore, it can be concluded that the *L. plantarum* HOM2217 strain may achieve the purpose of weight loss by reducing the expression of TNF-$\alpha$ and reducing inflammation.

It can be seen from the results in FIG. 15 that after oral administration of the test substance to obesity model rats for 49 days, compared with the negative control group, the contents of lactic acid, formic acid, acetic acid, propionic acid and butyric acid in the cecum of the model control group were reduced, and there was a significant difference (p < 0.05). Compared with the model control group, the medium-dose of *L. plantarum* HOM2217 significantly (p < 0.05) increased the content of formic acid in the cecum, the high-dose of *L. plantarum* HOM2217 significantly (p < 0.05) increased the content of lactic acid in the cecum, and extremely significantly (p < 0.01) increased the content of formic acid, acetic acid and butyric acid in the cecum.

In summary, after 49 days of administration of the *L. plantarum* HOM2217 strain, compared with the model control group, the tested substance at the medium-dose *L. plantarum* HOM2217 strain reduced body fat weight (p < 0.05) and fat/body weight ratio (p < 0.05), and increased serum high-density lipoprotein cholesterol (p < 0.05) in obesity model rats. High doses *L. plantarum* HOM2217 strain reduced body weight (p < 0.05), total weight gain (p < 0.01) and total food utilization rate (p < 0.01) in obesity model rats, increased serum high-density lipoprotein cholesterol (p < 0.01), decreased serum cytokines TNF-$\alpha$ levels (p < 0.01), and increased the levels of lactic acid (p < 0.05), formic acid (p < 0.01), acetic acid (p < 0.01), and butyric acid (p < 0.01) in the cecum. There was no significant difference in total food intake and total caloric intake of rats between each dose group and the model control group (p > 0.05). Therefore, it can be seen that, without affecting food intake, the *L. plantarum* HOM2217 strain achieved weight loss by producing short-chain fatty acids through metabolism, reducing the intestinal pH value, inhibiting the reproduction of obese flora, and reducing the expression of TNF-$\alpha$ to reduce the inflammatory response.

**Example 14. In Vivo Test 2 of *L. plantarum* HOM2217 Strain in Obesity Animal Model**

(1) Experimental Animals and Feed

85 healthy SPF-grade C57BL/6J (male, 4 weeks old, 15-17 g) mice propagated by Beijing Vital River Laboratory Animal Technology Co., Ltd were used as experimental animals.

The feed is selected from high-fat feed D12492 (60% fat content) and ordinary feed D12450B (10% fat content) produced byBeijing Keao Xieli Feed Co.,Ltd.

(2) Animal Model and Experimental Treatment

Animals were grouped after 1 week of acclimatization. After the end of the acclimatization, 68 mice were randomly divided into 2 groups according to body weight, wherein 8 mice were given maintenance feed as a negative control group, and 60 mice were given high-calorie model feed. After feeding for 14 days, the mice given with high-calorie model feed were ranked according to weight gain, 1/3 obesityresistant mice with lower weight gain were eliminated, and 40 obesity-sensitive mice were selected. They were randomly divided into 5 groups according to body weight: a model control group, a positive control group (Orlistat, 0.4 mg/mouse/day), a low-dose group (1 $\times$ 10$^9$CFU/mouse/day) of HOM2217 viable bacteria (using the powder of *L. plantarum* HOM2217 prepared in Example 11), a high-dose group ($5 \times 10^9$ CFU/mouse/day) of HOM2217 viable bacteria (using the powder of *L. plantarum* HOM2217 prepared in Example 11), and a dead bacteria group ($5 \times 10^9$ TFU/mouse/day) of HOM2217(using the powder of *L. plantarum* HOM2217 prepared in Example 12), with 8 mice in each group. Starting from the experiment, each animal in the model control group and each dosage group was given the same amount of high-calorie model feed daily, while the negative control group was given the same amount of maintenance feed in the same manner. Different dosage group was given different doses of the test substance, while the negative control group and the model control group were given sterile water. The test period was 11 weeks.

(3) Detection Indicators and Method

During the experiment, the feeding amount and remaining feeding amount of each cage of animals were recorded weekly, and the daily food intake amount and food intake calories of each mouse were calculated. The body weight of the mice was recorded weekly, and the weight gain and feed utilization rate were calculated.

$$\text{Feed utilization rate} = \text{weight gain} / \text{food intake} \times 100\%$$

(4) Data Processing

Statistical analysis was performed using SPSS software (ANOVA) and the Dennett method for pairwise comparisons of means between multiple experimental groups and a control group. The weight of rats in the negative control group and the model control group before and after the establishment of the obesity model was compared using t-test of independent samples.

(5) Experimental Results

The experimental results were shown in FIGS. 16-18.

It can be seen from the results in FIG. 16 that after 2 weeks of administration of high-calorie model feed, the weight of the mice increased by comparison between the model control group and the negative control group, with an extremely significant difference ($p < 0.01$), indicating that the model was successfully established. There was no significant difference in body weight between each dosage group and the model control group before administration of the test substance. After 11 weeks of oral administration of the test substance, compared with the negative control group, the weight of mice in the model control group increased, and there was a significant difference ($p < 0.01$). Compared with the model control group (average body weight of 33.9 g), the low-dose viable bacteria group of *L. plantarum* HOM2217 (average body weight of 29.8 g), the high-dose viable bacteria group of *L. plantarum* HOM2217 (average body weight of 29.1 g), the dead bacteria group of *L. plantarum* HOM2217 (average body weight of 30.4 g), and the positive control group (Orlistat) (average body weight of 29.2 g) all significantly reduced the final body weight of mice ($p < 0.01$), and the weight reduction rates were 12.1%, 14.2%, 10.3% and 13.9%, respectively. The reduction rate of the low-dose viable bacteria, the high-dose viable bacteria and the dead bacteria group of the *L. plantarum* HOM2217 on body weight was comparable to the effect of the positive control group (orlistat), with no significant difference ($p > 0.05$).

It can be seen from the results in FIG. 16 that after 11 weeks of administration of high-calorie model feed, the weight of mice increased by comparison between the model control group and the negative control group, with an extremely significant difference ($p < 0.01$), indicating that the model was successfully established. After 11 weeks of oral administration of the test substance, compared with the negative control group, the weight of mice in the model control group increased, and there was a significant difference ($p < 0.05$). Compared with the model control group (average body weight of 577 g), the high-dose of *L. plantarum* HOM2217(average body weight of 535.4 g) significantly reduced the final body weight of mice ($p < 0.05$), and the weight reduction rate was 7.2%.

It can be seen from the results in FIG. 17 that after the high-calorie model feed was given, compared with the negative control group, the total weight gain and the total food utilization rate of the mice in the model control group were both increased, and there was an extremely significant difference ($p < 0.01$). After 11 weeks of oral administration of the test substance, compared with the model control group, the low-dose viable bacteria group, the high-dose viable bacteria group, the dead bacteria group of *L. plantarum* HOM2217 and the positive control group (orlistat) group can significantly reduce the total weight gain of mice ($p < 0.01$). Compared with the model control group, both the high-dose viable bacteria group of *L. plantarum* HOM2217 and the positive control group (orlistat) group can significantly ($p < 0.05$) reduce the total food utilization rate of mice. The total weight gain of the low-dose viable bacteria, the high-dose viable bacteria and the dead bacteria group of *L. plantarum* HOM2217 was comparable to that of the positive control group (orlistat), and the difference was not significant ($p > 0.05$), and the total feed utilization rate of the high-dose viable bacteria group of the *L.*

*plantarum* HOM2217 was comparable to that of the positive control group (orlistat), and the difference is not significant (p > 0.05).

It can be seen from the results in FIG. 18 that after the high-calorie feed was administered, compared with the negative control group, the total food intake of the mice in the model control group was reduced and there was a significant difference (p < 0.05). After 11 weeks of oral administration of the test substance, there was no significant difference (p > 0.05) in total food intake and the total caloric intake of the mice between the experimental groups and the model control group.

In summary, after 11 weeks of administration of the *L. plantarum* HOM2217 strain, compared with the model control group, the low-dose viable bacteria group, the high-dose viable bacteria group and the dead group of *L. plantarum* HOM2217 can significantly reduce the body weight of obesity model mice (p < 0.01), reduce the total weight gain of obesity model mice (p < 0.01), and the weight loss effect was comparable to that of the positive control group (orlistat). There was no significant difference (p > 0.05) in the total intake and total caloric intake of mice between each dosage group and the model control group. Therefore, it can be seen that the *L. plantarum* HOM2217 strain has a weight loss effect without affecting food intake.

Although the embodiments of the present invention have been disclosed as above, they are not limited to the applications listed in the specification and the embodiments. They can be fully applied to various fields suitable for the present invention, and those skilled in the art can easily realize other modifications. Therefore, without departing from the general concept defined by the claims and the equivalent scope, the present invention is not limited to specific details and the details shown and described herein.

**Claims**

1. A *Lactiplantibacillus plantarum* (*L. plantarum*) strain, the *L. plantarum* strain is deposited in China General Microbiological Culture Collection Center (CGMCC) with a deposit number of CGMCC No. 25683.

2. The *L. plantarum* strain according to claim 1, wherein the *L. plantarum* strain comprises a 16S rRNA gene represented by SEQ ID NO: 1.

3. The *L. plantarum* strain according to claim 1, wherein the *L. plantarum* strain is isolated from healthy human breast milk.

4. A probiotic preparation, which is a viable bacteria preparation or a dead bacteria preparation, and comprises the *L. plantarum* strain according to claim 1.

5. The probiotic preparation according to claim 4, wherein the probiotic preparation is a viable bacteria preparation, and the viable bacteria content is 6 to $9 \times 10^{11}$ CFU/g.

6. The probiotic preparation according to claim 4, wherein the probiotic preparation is a dead bacteria preparation, and comprises 7 to $13 \times 10^{11}$ CFU/g of dead bacteria.

7. A method for preparing the probiotic preparation according to anyone of claims 4 to 6, comprising the following steps:

   (1) strain culturing: inoculating the *L. plantarum* strain into a sterile liquid medium at an inoculation amount of 1 to 3% based on the total amount of the medium, culturing for 16 to 24 hours to obtain a seed culture solution, then inoculating the obtained seed culture solution into a fermentation medium at an inoculation amount of 1 to 3% based on the total amount of the medium for fermentation culture to obtain a high-density culture solution of *L. plantarum*;
   (2) drying.

8. The method according to claim 7, wherein the fermentation medium used comprises: glucose 60 to 100 g/L, yeast extract 60 to 80 g/L, magnesium sulfate 0.2 to 0.4 g/L, manganese sulfate 0.1 to 0.2 g/L, dipotassium hydrogen phosphate 2 to 4 g/L, potassium dihydrogen phosphate 2 to 4 g/L, Tween-80 1 to 2 g/L, and L-cysteine salt 0.5 to 2 g/L.

9. The method according to claim 7, wherein during the fermentation, the sodium hydroxide solution is automatically added to maintain a constant pH of 5.0 to 6.0, so that the fermentation is terminated until the acid production is stopped, and the fermentation is terminated when the sodium hydroxide is no longer added.

10. The method according to anyone of claims 7 to 9, wherein the probiotic preparation is a viable bacteria preparation, and the method further comprises preparing a lyoprotectant after the strain culturing step and before the drying step, and performing the freeze-drying in the drying step; optionally, the lyoprotectant used comprises: protectant of 50 to 100 g/L peptone, 20 to 60 g/L sucrose, 5 to 10 g/L vitamin C and 1 to 3 g/L glycerol.

11. The method according to claim 10, wherein the conditions of freeze-drying are: a pre-freezing temperature of -50 to -55 °C with pre-freezing time of 5 to 8 hours, a primary drying temperature of -10 to -30 °C with primary drying time of 25 to 35 hours, and a secondary drying temperature of 32 to 35 °C with secondary drying time of 5 to 8 hours.

12. The method according to anyone of claims 7 to 9, wherein the probiotic preparation is a dead bacteria preparation, and the drying step is to perform heat inactivation first, and then drying through a spray drying tower to obtain the dead bacteria preparation; optionally, the heat inactivation is performed at 70 to 100 °C for 10 to 40 minutes.

13. Use of the *L. plantarum* strain according to any one of claims 1 to 3 for the production of lactic acid and short-chain fatty acids.

14. The use according to claim 13, wherein the short-chain fatty acid is selected from the group consisting of one or more of formic acid, acetic acid, propionic acid, butyric acid and isobutyric acid.

15. Use of the *L. plantarum* strain according to any one of claims 1 to 3 in the preparation of a composition for inhibiting the obese strain *Enterobacter cloacae*.

16. Use of the *L. plantarum* strain according to any one of claims 1 to 3 in the preparation of a composition for inhibiting the differentiation of 3T3-L1 preadipocytes, thereby effectively inhibiting fat deposition.

17. Use of the *L. plantarum* strain according to anyone of claims 1 to 3 in the preparation of a pharmaceutical composition for weight loss, or reducing hyperlipidemia and hypercholesterolemia caused by obesity.

18. The use according to claim 17, wherein the weight loss function is to reduce the content of cholesterol, or inhibit the differentiation of 3T3-L1 preadipocytes, thereby inhibiting fat production.

19. The use according to claim 17, wherein the weight loss function is one or more selected from the group consisting of reducing body weight, total weight gain and total food utilization rate in obesity model rats; reducing body fat weight and fat/body weight ratio in obesity model rats; increasing serum high-density lipoprotein cholesterol content; increasing intestinal short-chain fatty acid content; and reducing the expression level of cellular inflammatory factor TNF-$\alpha$ in serum.

20. The use according to any one of claims 17 to 19, wherein the weight loss function is one or more selected from the group consisting of reducing cholesterol content, inhibiting differentiation of 3T3-L1 preadipocytes, reducing body weight, total weight gain and total food utilization rate in obesity model rats.

FIG. 1

Sorensen's Coefficient

FIG. 2

FIG. 3

FIG. 4

FIG. 5

FIG. 6

FIG. 7

FIG. 8

Group

FIG. 9

Group

FIG. 10

Group

FIG. 11

Group

FIG. 12

Group

FIG. 13

Group

FIG. 14

FIG. 15

Group

FIG. 16

Group

FIG. 17

Group

FIG. 18

## INTERNATIONAL SEARCH REPORT

| International application No. |
|---|
| **PCT/CN2024/100581** |

| A. | CLASSIFICATION OF SUBJECT MATTER |
|---|---|

C12N1/20(2006.01)i;  A61K35/747(2015.01)i;  A61P3/06(2006.01)i;  A61P31/04(2006.01)i;  C12R1/25(2006.01)i;
C12P7/56(2006.01)i;  A61P3/04(2006.01)i

According to International Patent Classification (IPC) or to both national classification and IPC

| B. | FIELDS SEARCHED |
|---|---|

Minimum documentation searched (classification system followed by classification symbols)

IPC: C12N, A61K, A61P, C12R, C12P

Documentation searched other than minimum documentation to the extent that such documents are included in the fields searched

Electronic data base consulted during the international search (name of data base and, where practicable, search terms used)

VEN, CNABS, PubMed, DWPI, CNTXT, WOTXT, USTXT, EPTXT, JPTXT, CNKI, Web of Science, 百度学术, BAIDU
SCHOLAR, Patentics, 中国生物序列检索系统, China Patents Biological Sequence Search System, NCBI Genbank, EBI,
STNext: 申请人、发明人、植物乳植杆菌, Lactiplantibacillus plantarum, 植物乳杆菌, Lactobacillus plantarum, CGMCC
No.25683, HOM2217, 肥胖, obesity, 序列1

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 117327623 A (COREE CO., LTD.) 02 January 2024 (2024-01-02)<br>claims 1-23 | 1-20 |
| PX | CN 117327622 A (COREE CO., LTD.) 02 January 2024 (2024-01-02)<br>claims 1-11 | 1-20 |
| Y | CN 116064333 A (BEIJING SANYUAN FOODS CO., LTD.) 05 May 2023 (2023-05-05)<br>abstract, and embodiment 2 | 1-20 |
| Y | CN 116179419 A (GUANGXI UNIVERSITY) 30 May 2023 (2023-05-30)<br>abstract, and description, paragraphs 4-5 | 1-20 |
| Y | CN 116144541 A (GUANGXI AISHENG LIFE TECHNOLOGY CO., LTD.) 23 May 2023<br>(2023-05-23)<br>abstract | 15 |

| ✓ | Further documents are listed in the continuation of Box C. | ✓ | See patent family annex. |
|---|---|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **29 September 2024** | **10 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/100581** |

**C. DOCUMENTS CONSIDERED TO BE RELEVANT**

| Category* | Citation of document, with indication, where appropriate, of the relevant passages | Relevant to claim No. |
| --- | --- | --- |
| Y | 陆婧婧等 (LU, Jingjing et al.). "植物乳杆菌KLDS 1.0344体外抗氧化特性及抑制脂肪沉积的研究 (Antioxidant Properties and Inhibition of Fat Deposition of Lactobacillus Plantarum KLDS 1.0344 in Vitro)" 食品科技 (Food Science and Technology), Vol. vol. 43, No. 10, 31 December 2018 (2018-12-31), pp. 1-7 | 16 |
| Y | CN 112812999 A (GUANGXI AISHENG LIFE TECHNOLOGY CO., LTD.) 18 May 2021 (2021-05-18) abstract | 17-20 |
| A | WO 2023088591 A1 (PROBITAT OY) 25 May 2023 (2023-05-25) abstract | 1-20 |
| A | CN 116790432 A (AUSNUTRIA DAIRY (CHINA) CORP., LTD.) 22 September 2023 (2023-09-22) abstract | 1-20 |
| A | Fang, S. "Lactiplantibacillus Plantarum Strain 7430 16S Ribosomal RNA Gene, Partial Sequence" GenBank: MT516067.1, 01 June 2020 (2020-06-01), sequence part | 1-20 |

Form PCT/ISA/210 (second sheet) (July 2022)

**INTERNATIONAL SEARCH REPORT**

International application No.

**PCT/CN2024/100581**

| **Box No. I** | **Nucleotide and/or amino acid sequence(s) (Continuation of item 1.c of the first sheet)** |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.

**PCT/CN2024/100581**

| Patent document cited in search report | | | Publication date (day/month/year) | Patent family member(s) | | | Publication date (day/month/year) |
|---|---|---|---|---|---|---|---|
| CN | 117327623 | A | 02 January 2024 | None | | | |
| CN | 117327622 | A | 02 January 2024 | None | | | |
| CN | 116064333 | A | 05 May 2023 | None | | | |
| CN | 116179419 | A | 30 May 2023 | None | | | |
| CN | 116144541 | A | 23 May 2023 | None | | | |
| CN | 112812999 | A | 18 May 2021 | None | | | |
| WO | 2023088591 | A1 | 25 May 2023 | AU | 2022392707 | A1 | 30 May 2024 |
| | | | | ES | 2965365 | T3 | 15 April 2024 |
| | | | | EP | 4036219 | A1 | 03 August 2022 |
| | | | | EP | 4036219 | C0 | 08 November 2023 |
| CN | 116790432 | A | 22 September 2023 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311294650
- CN 202311294687X

**Non-patent literature cited in the description**

- *Report on the Nutrition and Chronic Disease Status of Chinese Residents*, 2020
- National Food Safety Standard - Determination of Cholesterol in Food. *GB 5009.128-2016*